(19) **Europäisches Patentamt**
**European Patent Office**
**Office européen des brevets**

(11) **EP 3 849 991 B1**

(12) **EUROPÄISCHE PATENTSCHRIFT**

(45) Veröffentlichungstag und Bekanntmachung des Hinweises auf die Patenterteilung:
**20.08.2025 Patentblatt 2025/34**

(21) Anmeldenummer: **19765728.1**

(22) Anmeldetag: **10.09.2019**

(51) Internationale Patentklassifikation (IPC):
**C07H 1/00** (2006.01)     **C07H 15/04** (2006.01)
**A23L 29/30** (2016.01)     **A23L 27/30** (2016.01)

(52) Gemeinsame Patentklassifikation (CPC):
**C07H 1/00; A23L 27/34; C07H 15/04**

(86) Internationale Anmeldenummer:
**PCT/EP2019/074102**

(87) Internationale Veröffentlichungsnummer:
**WO 2020/053207 (19.03.2020 Gazette 2020/12)**

(54) **VERFAHREN ZUR VERBESSERTEN HERSTELLUNG VON ISOMALT**

METHOD FOR IMPROVED PRODUCTION OF ISOMALT

PROCÉDÉ DE FABRICATION AMÉLIORÉE D' ISOMALT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.09.2018 EP 18193756**

(43) Veröffentlichungstag der Anmeldung:
**21.07.2021 Patentblatt 2021/29**

(73) Patentinhaber: **Südzucker AG**
**68165 Mannheim (DE)**

(72) Erfinder:
• **HAJI BEGLI, Alireza**
**67305 Ramsen (DE)**

• **KRÖNER, Christine**
**67271 Kindenheim (DE)**
• **CARTARIUS, Ralph**
**74254 Offenau (DE)**

(74) Vertreter: **Schrell, Andreas et al**
**Gleiss Große Schrell und Partner mbB**
**Patentanwälte Rechtsanwälte**
**Leitzstrasse 45**
**70469 Stuttgart (DE)**

(56) Entgegenhaltungen:
**WO-A1-2005/021475     WO-A1-2011/076625**
**WO-A2-03/104473     DE-T2- 69 613 100**

# EP 3 849 991 B1

**Beschreibung**

Die vorliegende Erfindung betrifft ein Verfahren zur verbesserten Herstellung von Isomalt, insbesondere eines besonders lagerstabilen und reinen Isomalts, aus einer Isomaltulose- oder Isomaltulose- und Trehalulose- sowie Saccharose-haltigen Kohlenhydratmischung, welches sich insbesondere durch verbesserte Effizienz, Prozessstabilität und Reaktionsselektivität sowie - ausbeute auszeichnet, und das so hergestellte Isomalt.

Isomalt (hydrierte Isomaltulose) ist ein Zuckeraustauschstoff, der als Hauptbestandteile 1,6-GPS (6-O-$\alpha$-D-Glucopyranosyl-D-sorbit) und 1,1-GPM (1-O-$\alpha$-D-Glucopyranosyl-D-mannit) aufweist. Aufgrund seiner nicht-kariogenen, zahnfreundlichen Eigenschaften, seines geringen Brennwerts und seiner niedrigen glykämischen Wirkung besitzt Isomalt zahlreiche ernährungsphysiologische Vorteile. Gemäß der Joint FAO/WHO Expert Committee on Food Additives (JECFA) Spezifikation (69. JECFA (2008), publiziert in FAO JECFA Monographs 5 (2008)), weist Isomalt mindestens 98 Gew.-% hydrierte Mono- und Disaccharide (nämlich 1,6-GPS, 1,1-GPM, 1,1-GPS (1-O-$\alpha$-D-Glucopyranosyl-D-sorbit), Sorbit und Mannit) und höchstens 2 Gew.-% Nebenkomponenten auf, wobei dieses Isomalt mindestens 86 Gew.-% 1,6-GPS und 1,1-GPM und 0 bis 0,3 Gew.-% reduzierende Zucker enthält (jeweils basierend auf Trockensubstanz des Isomalts). Ein lebensmittelrechtlich als "Zuckerfrei" geltendes Isomalt gemäß JECFA-Spezifikation, das zusätzlich den europäischen Reinheitskriterien für Zusatzstoffe und Anforderungen aus Standardwerken, wie dem FCC (Food Chemical Codex) sowie einschlägigen Isomaltmonographien der Arzneimittelbücher in Europa, den USA oder Japan gerecht wird, ist darüber hinaus dadurch ausgezeichnet, dass einzelne der gegebenenfalls vorhandenen Nebenkomponenten jeweils höchstens in einer Menge von 0,5 Gew.-% vorliegen und dass insgesamt in dem Isomalt höchstens 0,5 Gew.-% reduzierende und nicht-reduzierende Zucker (zum Beispiel Mono- und Disaccharide) (jeweils basierend auf Trockensubstanz des Isomalts) vorliegen.

Isomalt ist in verschiedenen Varianten bekannt. So kann, wie aus zum Beispiel DE 195 32 396 C2 ersichtlich, Isomalt als nahezu äquimolares Gemisch von 1,6-GPS und 1,1-GPM vorliegen.

Andere Isomaltvarianten zeichnen sich durch zum Beispiel einen erhöhten 1,6-GPS-Gehalt aus (sogenanntes 1,6-GPS-angereichertes Isomalt), insbesondere dadurch, dass ein 1,6-GPS-Gehalt von 57 bis 99 Gew.-% und ein 1,1-GPM-Anteil von 43 bis 1 Gew.-% (bezogen auf die Isomalthauptbestandteile) vorliegt und sind aufgrund dieser Zusammensetzung mit einer erhöhten Süßkraft und Löslichkeit ausgestattet. Wie aus DE 195 23 008 A1 oder der EP 2 361 255 B1 bekannt, lassen sich derartige 1,6-GPS-angereicherte Gemische unter Verwendung von Rutheniumkatalysatoren herstellen.

Die Herstellung von Isomalt erfolgt in der Regel in einem zweistufigen Prozess, in dem zunächst Isomaltulose (auch bekannt als Palatinose) aus Saccharose Enzym-katalytisch mittels Saccharose-Glucosylmutasen gewonnen und anschließend die Isomaltulose Metall-katalytisch mit Wasserstoff ($H_2$) hydriert wird.

Bei der enzymatischen Isomerisierung der Saccharose zu Isomaltulose wird aus der 1,2-glykosidischen Verknüpfung von Glucose und Fructose in der Saccharose eine 1,6-glykosidische Verknüpfung in der Isomaltulose. Dadurch wird aus der nicht-reduzierenden Saccharose, die keine freie Aldehyd- oder Keto-Gruppe enthält, die reduzierende Isomaltulose, ein Keto-Disaccharid mit freier Keto-Gruppe. Die 1,6-glykosidische Verknüpfung zwischen Glucose und Fructose in der Isomaltulose ist, im Vergleich zu der 1,2-glykosidischen Bindung in der Saccharose, erheblich Hydrolyse-stabiler, wodurch die Isomaltulose erheblich resistenter gegenüber Säuren und mikrobieller Fermentation ist.

Im industriellen Maßstab erfolgt die Herstellung von Isomalt, wie aus der EP 0 625 578 A1 ersichtlich, ausgehend von Saccharose oder einer Saccharose-haltigen Ausgangsmischung, indem die Saccharose oder die Saccharose-haltige Ausgangsmischung einer enzymatischen Isomerisierungsreaktion unter Einsatz einer Saccharose-Glucosylmutase unterworfen wird, die zur Bildung einer sogenannten "isomerisierten Saccharose" führt, in der, in aller Regel, neben der Isomaltulose auch nicht umgesetzte Restmengen an Saccharose, auch als "Restsaccharose" bezeichnet, enthalten sind. Diese aus der Isomerisierung der Saccharose oder Saccharose-haltigen Ausgangsmischung erhaltene "isomerisierte Saccharose", im Folgenden auch als Isomaltulose- und Saccharose-haltige Kohlenhydratmischung bezeichnet, wird sodann einer Hydrierreaktion unterzogen, um die Isomaltulose und gegebenenfalls in der Kohlenhydratmischung auch vorliegende, durch eine andere Isomerisierung der Saccharose entstandene, Trehalulose zu den Disaccharidalkoholen 1,6-GPS und 1,1-GPM sowie gegebenenfalls 1,1-GPS (1-O-$\alpha$-D-Glucopyranosyl-D-sorbit, aus Trehalulose) zu reduzieren.

Wird die Hydrierung unter üblichen Prozessbedingungen in neutralem oder alkalischem Milieu, in der Regel unter Verwendung von Raney-Nickel-Katalysatoren (EP 0 625 578 A), durchgeführt, bleibt die in der "isomerisierten Saccharose" vorhandene Restsaccharosemenge unverändert, da die Saccharose aufgrund ihrer chemischen Struktur nicht reduzierend ist und weder hydrolytisch gespalten, noch hydriert werden kann. Eine unmittelbare Herstellung eines Isomalts mit einem im Vergleich zum 1,1-GPM-Gehalt höheren 1,6-GPS-Gehalt ist mittels einer solchen Verfahrensweise allerdings nicht ohne weiteres möglich. Findet eine Hydrierung von Saccharose allerdings unter üblichen Prozessbedingungen im sauren Milieu statt, wird - wie aus der US 4,072,628, US 3,963,788, oder US 4,950,812 ersichtlich, in denen die Hydrierung von Saccharose unter Verwendung von Ruthenium-basierten Katalysatoren offenbart ist - die Säurelabile Saccharose zu Glucose und Fructose hydrolysiert, die als reduzierende Zucker zu Sorbit und Mannit hydriert werden. Man spricht in diesem Fall von einer "spaltenden Hydrierung" der Saccharose. Die Isomaltulose bleibt dagegen aufgrund ihrer hohen Säurestabilität auch unter sauren Hydrierbedingungen stabil und kann, wie aus der DE 195 23 008 A1 hervorgeht, ohne Weiteres direkt zu Isomalt hydriert werden, insbesondere unter Nutzung eines Ruthenium-basierten Katalysators.

Aus der EP 2 361 255 B1 ist bekannt, eine Mischung aus Isomaltulose und "Restsaccharose" mittels eines Ruthenium-basierten Katalysators zur Herstellung von Isomalt zu hydrieren, wobei die Saccharose unter die Saccharose-Hydrolyse ermöglichenden Bedingungen über Glucose und Fructose zu Sorbit und Mannit spaltend hydriert und die Isomaltulose zu 1,1-GPM und 1,6-GPS hydriert wird. Die Verwendung eines Ruthenium-basierten Katalysators führt insbesondere zur Herstellung eines Isomalts mit einem im Vergleich zum 1,1-GPM-Gehalt höheren 1,6-GPS-Gehalt, was für bestimmte Anwendungen von Vorteil ist (DE 195 23 008 A1). Ein derartig hergestelltes Isomalt weist aufgrund der in den beschriebenen Ruthenium-basierten Verfahren eingesetzten Verfahrensbedingungen allerdings eine für viele Anwendungen unerwünschte große Anzahl und Menge von Nebenprodukten auf. Unter den in der Druckschrift dargestellten konkreten Verfahrensbedingungen der Beispiele 1 bis 4, insbesondere Temperaturen von 90 und 120 °C sowie einem Druck von 60 bar, wird die Isomaltulose nur mit vergleichsweise geringer Umsatzrate umgesetzt oder es werden energetisch ungünstige hohe Temperaturen eingesetzt. Zudem ist die Standzeit des eingesetzten Katalysators verkürzt, was zu erhöhten Produktionskosten führt. Darüber hinaus setzt die Anwesenheit dieser Nebenprodukte auch die Reinheit des erhaltenen Produktes und damit deren Verkehrsfähigkeit herab. So ist zwar einerseits die im benannten Verfahren erzielte Spaltung der Saccharose zu Glucose und Fructose und die damit einhergehende Hydrierung zu den Nebenprodukten Sorbit und Mannit insofern wünschenswert, als dass aufgrund ernährungsphysiologischer Anforderungen unerwünschte größere Mengen an Rest-Saccharose im bereitzustellenden Isomalt vermieden werden. Andererseits sind die aus der spaltenden Hydrierung erhaltenen Monosaccharidalkohole Sorbit und Mannit, insbesondere Sorbit, technologisch häufig nachteilig, da sie aufgrund ihrer Hygroskopizität gegebenenfalls notwendig werdende Trocknungsschritte im Isomaltherstellverfahren erschweren, zumindest aber teurer machen und deren Anwesenheit in manchen Anwendungen, beispielsweise in Hartkaramellen, aufgrund ihrer Hygroskopizität zu unerwünschten Produkteigenschaften wie Klebrigkeit, schlechter Formstabilität, wie ein Zerfließen der Produkte (EP 1 776 015 B1), und schlechter Lagerfähigkeit führt.

Insbesondere in Fällen, in denen - in der Regel im industriellen Maßstab enzymatisch aus Sacharose oder Saccharose-haltigen Ausgangsmischungen gewonnene - Saccharose-haltige Isomaltulose-Gemische mit einem sehr geringen Restsaccharose-Gehalt zu Isomalt hydriert werden sollen, kann es daher wünschenswert sein, dass die Saccharose das Hydrierverfahren unverändert passiert, sodass aus dieser kein Sorbit und Mannit gebildet wird.

Die erhöhte Hygroskopizität der hydrierten Spaltungsprodukte von Saccharose, insbesondere Sorbit, im Verhältnis zu Saccharose selbst sowie zu Isomalt, bewirkt darüber hinaus eine Verringerung der Lagerfähigkeit des Isomalts, insbesondere in feuchten und heißen Klimaregionen, und erschwert die Verwendung von Isomalt in besagten feuchten und heißen Klimaregionen, insbesondere in Süßigkeiten, beispielsweise Hartkaramellen, und insbesondere in Medikamenten, welche eine aufwendige Verpackung benötigen.

DE 696 13 100 T2, WO 2005/021475 A1 und WO 03/104473 A2 offenbaren Verfahren zur Herstellung von Zuckeralkoholen aus verschiedenen Zucker-haltigen Ausgangspräparationen.

Obgleich eine Reihe von Verfahren zur Herstellung von Isomalt aus Isomaltulose- und Saccharose-haltigen Kohlenhydratmischungen bekannt sind, die durch enzymatische Umsetzung von Saccharose oder Saccharose-haltigen Ausgangsmischungen gewonnen wurden, besteht nach wie vor ein Bedarf an Verfahren, die Isomalt, insbesondere 1,6-GPS-angereichertes Isomalt, in hoher Reinheit und mit für bestimmte Anwendungsgebiete optimiertem Eigenschaftsprofil aus Isomaltulose- und Saccharose-haltige Kohlenhydratmischungen bereitstellen, welche vorzugsweise aus Saccharose oder Saccharose-haltigen Ausgangsmischungen enzymatisch gewonnen wurden.

Der vorliegenden Erfindung liegt das technische Problem zugrunde, die vorstehenden Nachteile zu überwinden, insbesondere ein besonders prozessstabiles, kostengünstiges Verfahren zur Herstellung von Isomalt, insbesondere solchem, das einen höheren 1,6-GPS- als 1,1-GPM-Anteil aufweist, aus einer, vorzugsweise aus einer Saccharose-Glucosylmutase-katalysierten Umsetzung von Saccharose oder Saccharose-haltigen Ausgangsmischungen gewonnenen, Isomaltulose- und Saccharose-haltigen Kohlenhydratmischung bereitzustellen, welches ein besonders lagerstabiles Isomalt, insbesondere für die Anwendung in Süßigkeiten, beispielsweise Hartkaramellen, effizient in hoher Reinheit der Hauptkomponenten 1,6-GPS und 1,1-GPM und in hoher Ausbeute bereitstellt. Die Erfindung stellt auch ein mittels dieses Verfahrens bereitgestelltes besonders reines Isomalt bereit, insbesondere ein solches, das als "zuckerfrei" bezeichnet werden kann, insbesondere also einen Zuckergehalt, insbesondere Saccharosegehalt, von höchstens 0,50 Gew.- % (bezogen auf TS des Isomalts) aufweist und sich gleichzeitig durch besonders geringe Sorbit- und Mannitgehalte auszeichnet.

Die vorliegende Erfindung löst das ihr zugrundeliegende technische Problem durch die Bereitstellung der vorliegenden technischen Lehre, insbesondere der Lehre der unabhängigen Ansprüche und der in der Beschreibung sowie den abhängigen Ansprüchen offenbarten bevorzugten Ausführungsformen.

Erfindungsgemäß bereitgestellt wird ein Verfahren zur kontinuierlichen Herstellung von Isomalt aus einer Isomaltulose- und Saccharose-haltigen Kohlenhydratmischung, umfassend die Verfahrensschritte

a) Bereitstellung einer in wässrigem Medium vorliegenden Isomaltulose- und Saccharose-haltigen Kohlenhydratmischung, enthaltend 75,00 bis 99,99 Gew.-% Isomaltulose und 0,01 bis 0,50 Gew.-% Saccharose (jeweils TS (Trockensubstanz), bezogen auf Gesamt-TS der Kohlenhydratmischung), von Wasserstoff und eines Ruthenium-basierten Katalysators,

b) Umsetzung der Kohlenhydratmischung zu Isomalt durch kontinuierliches Inkontaktbringen der im wässrigen Medium vorliegenden Kohlenhydratmischung mit dem Ruthenium-basierten Katalysator und Wasserstoff mit einer Raumgeschwindigkeit von 0,25 bis 1,5 h$^{-1}$, bei einem Wasserstoffdruck von 16,0 bis 22,0 MPa und einem pH-Wert von 2,0 bis 6,0 zum Erhalt eines Isomalt-haltigen Produktstroms unter Einstellen einer Reaktionstemperatur von höchstens 100 °C und

c) Erhalt des Isomalts.

Die vorliegende Verfahrensweise zeichnet sich demgemäß dadurch aus, dass in einem ersten Verfahrensschritt a) eine Isomaltulose- und Saccharose-haltige Kohlenhydratmischung in wässrigem Medium enthaltend 75,00 bis 99,99 Gew.-% Isomaltulose sowie 0,01 bis 0,50 Gew.-% Saccharose, Wasserstoff sowie ein Ruthenium-basierter Katalysator, insbesondere ein geträgerter Katalysator, bereitgestellt werden und in einem zweiten Verfahrensschritt b) die im wässrigem Medium vorliegende Kohlenhydratmischung mit dem Ruthenium-basierten Katalysator und Wasserstoff in einer kontinuierlichen Verfahrensweise in Kontakt gebracht wird, sodass ein Reaktionsmedium erhalten wird, in welchem die Umsetzung der Kohlenhydratmischung zu Isomalt erfolgt. Erfindungsgemäß wird die Kohlenhydratmischung mit dem Ruthenium-basierten Katalysator und Wasserstoff in Kontakt gebracht bei einer Raumgeschwindigkeit von 0,25 bis 1,5 h$^{-1}$, bei einem Wasserstoffdruck von 16,0 bis 22,0 MPa (160 bis 220 bar), bei einem pH-Wert von 2,0 bis 6,0 und bei einer Temperatur von höchstens 100 °C, wobei vorzugsweise die Isomaltulose in Schritt b) mit einer Umsatzrate von 99 bis 100 mol-% und einer Selektivität von 97 bis 100 mol- % zu 1,6-GPS (6-O-$\alpha$-D-Glucopyranosyl-D-sorbit) und 1,1-GPM (1-O-$\alpha$-D-Glucopyranosyl-D-mannit) umgesetzt wird, um so in einem dritten Verfahrensschritt c) das Isomalt zu erhalten.

Die erfindungsgemäße Verfahrensweise zeichnet sich insbesondere und in vorteilhafter Weise vorzugsweise dadurch aus, dass unter Nutzung der in den Verfahrensschritten a) und b) definierten Merkmale, insbesondere der quantitativ definierten Isomaltulose- und Saccharose-haltigen Kohlenhydratmischung als Ausgangsmaterial und der in Verfahrensschritt b) angegebenen Verfahrensparameter eine Verfahrensweise bereitgestellt wird, die zu einer Umsatzrate der Isomaltulose von 99 bis 100 mol-% und einer Selektivität der Umsetzung von Isomaltulose zu 1,6-GPS und 1,1-GPM von 97 bis 100 mol-% führt.

Erfindungsgemäß bevorzugt bereitgestellt wird daher ein Verfahren zur kontinuierlichen Herstellung von Isomalt aus einer Isomaltulose- und Saccharose-haltigen Kohlenhydratmischung, umfassend die Verfahrensschritte

a) Bereitstellung einer in wässrigem Medium vorliegenden Isomaltulose- und Saccharose-haltigen Kohlenhydratmischung, enthaltend 75,00 bis 99,99 Gew.-% Isomaltulose und 0,01 bis 0,50 Gew.-% Saccharose (jeweils TS (Trockensubstanz), bezogen auf Gesamt-TS der Kohlenhydratmischung), von Wasserstoff und eines Ruthenium-basierten Katalysators,

b) Umsetzung der Kohlenhydratmischung zu Isomalt durch kontinuierliches Inkontaktbringen der im wässrigen Medium vorliegenden Kohlenhydratmischung mit dem Ruthenium-basierten Katalysator und Wasserstoff mit einer Raumgeschwindigkeit von 0,25 bis 1,5 h$^{-1}$, bei einem Wasserstoffdruck von 16,0 bis 22,0 MPa und einem pH-Wert von 2,0 bis 6,0 zum Erhalt eines Isomalt-haltigen Produktstroms unter Einstellen einer Reaktionstemperatur von höchstens 100 °C, wobei die Isomaltulose in Schritt b) mit einer Umsatzrate von 99 bis 100 mol-% und einer Selektivität von 97 bis 100 mol-% zu 1,6-GPS (6-O-$\alpha$-D-Glucopyranosyl-D-sorbit) und 1,1-GPM (1-O-$\alpha$-D-Glucopyranosyl-D-mannit) umgesetzt wird und

c) Erhalt des Isomalts.

Die Erfindung sieht daher vor, als Ausgangsmaterial für das erfindungsgemäß bereitgestellte Verfahren eine Kohlenhydratmischung enthaltend Isomaltulose einzusetzen, die sogenannte "Restsaccharose", also einen vergleichsweise geringen Anteil an Saccharose, nämlich 0,01 bis 0,50 Gew.-% Saccharose aufweist. Durch Einstellung der erfindungsgemäß vorgesehenen Verfahrensparameter ist es bei Verwendung eines derartigen Ausgangsmaterials erfindungsgemäß überraschenderweise in bevorzugter Ausführungsform möglich, ein Produkt besonders hoher Reinheit mit den nachstehend beschriebenen Vorteilen zu erreichen. Erfindungsgemäß ist es vorgesehen, das vorstehend definierte, mit einem geringen Anteil an Saccharose ausgestattete Ausgangsmaterial enthaltend Isomaltulose mit einer genau definierten Raumgeschwindigkeit von 0,25 bis 1,5 h$^{-1}$, bei einem Wasserstoffdruck von 16,0 bis 22,0 MPa und einem pH-Wert von 2,0 bis 6,0 mit dem Ruthenium-basierten Katalysator und Wasserstoff in Kontakt zu bringen, um so einen Isomalt-haltigen Produktstrom zu erhalten, wobei die Reaktionstemperatur dieses Isomalt-haltigen Produktstroms höchstens 100 °C betragen darf. Insbesondere ist die Reaktionstemperatur des Isomalt-haltigen Produktstroms so einzustellen, dass eine Isomaltulose-Umsatzrate von 99 bis 100 mol-% bei einer Selektivität von 97 bis 100 mol-% zu 1,6-GPS und 1,1-GPM erreicht wird.

Die erfindungsgemäß eingesetzten Verfahrensbedingungen sind besonders schonend und führen nur in sehr geringem Maße zur Bildung von, aus lebensmittelrechtlicher und anwendungstechnischer Sicht, unerwünschten Nebenprodukten. Insbesondere wurde überraschenderweise festgestellt, dass die in der eingesetzten Kohlenhydratmischung vorhandene Saccharose trotz der sauren Hydrierbedingung nicht gespalten und nicht hydriert wird. Vorteilhafterweise kann daher eine in vielen Anwendungsfällen unerwünschte Bildung von Sorbit und Mannit aus in der Kohlenhydratmischung vorhandener Saccharose vermieden werden. Da sich während des erfindungsgemäßen Verfahrens aus der eingesetzten Saccharose kein Sorbit und Mannit bildet, kann in Fällen, in denen im erhaltenen Isomalt aus zum Beispiel anwendungstechnischen Gründen eine definierte Höchstmenge von Sorbit und Mannit erwünscht ist, die Menge an gegebenenfalls vorhandener Glucose und Fructose in der für die Hydrierung eingesetzten Isomaltulose- und Saccharose-haltigen Kohlenhydratmischung entsprechend größer sein.

Die erfindungsgemäß vorgesehene Verfahrensweise, eine Höchsttemperatur von 100 °C im Zusammenspiel mit den weiteren Merkmalen der erfindungsgemäßen Lehre während der Umsetzung der Kohlenhydratmischung zu Isomalt einzusetzen, reduziert die Energiekosten und stellt in einem kostengünstigen Verfahren effizient und schnell vorteilhaftes Isomalt bereit.

Durch das erfindungsgemäße Verfahren kann in bevorzugter Ausführungsform ein Isomalt hergestellt werden, das der JECFA-Spezifikation genügt und im Folgenden auch als JECFA-gemäßes Isomalt bezeichnet wird. Ein derartiges Isomalt zeichnet sich durch einen Gehalt an mindestens 98 Gew.-% hydrierter Mono- und Disaccharide, nämlich 1,6-GPS, 1,1-GPM, 1,1-GPS, Sorbit und Mannit, und höchstens 2 Gew.-% Nebenkomponenten aus, wobei dieses Isomalt mindestens 86 Gew.-% 1,6-GPS und 1,1-GPM sowie 0 bis 0,3 Gew.-% reduzierende Zucker (zum Beispiel Glucose und Fructose) aufweist und wobei in diesem Isomalt höchstens 0,5 Gew.- %, insbesondere 0,01 bis 0,50 Gew.-%, insbesondere 0,01 bis 0,49 Gew.-%, insbesondere 0,01 bis 0,05 Gew.-% Saccharose (jeweils bezogen auf Gesamtgewicht der Trockensubstanz des Isomalts) vorhanden sind.

Durch das erfindungsgemäße Verfahren kann in besonders bevorzugter Ausführungsform auch ein Isomalt hergestellt werden, das ein besonders reines Isomalt ist, hier auch als hochreines Isomalt bezeichnet. Das erfindungsgemäß in bevorzugter Ausführungsform hergestellte hochreine Isomalt entspricht der Joint FAO/WHO Expert Committee on Food Additives Spezifikation (69. JECFA (2008), publiziert in FAO JECFA Monographs 5 (2008)) und ist demgemäß ein JECFA-gemäßes Isomalt mit einem Gehalt an mindestens 98,00 Gew.-% hydrierter Mono- und Disaccharide, nämlich 1,6-GPS, 1,1-GPM, 1,1-GPS, Sorbit und Mannit, und höchstens 2,00 Gew.-% Nebenkomponenten, wobei in dem Isomalt mindestens 98,00 Gew.- % 1,6-GPS und 1,1-GPM, 0 bis 0,50 Gew.-% Sorbit, 0 bis 0,50 Gew.-% Mannit und 0 bis 0,30 Gew.-% reduzierende Zucker (zum Beispiel Glucose und Fructose) vorhanden sind, wobei einzelne der in diesem Isomalt gegebenenfalls vorhandenen 2 Gew.-% Nebenkomponenten jeweils in einer Menge von 0 bis 0,50 Gew.-% vorhanden sind und wobei insgesamt höchstens 0,50 Gew.-% reduzierende und nicht-reduzierende Zucker (wie Isomaltulose, Isomaltose, Saccharose, Glucose oder Fructose), und höchstens 0,5 Gew.-%, insbesondere 0,01 bis 0,50 Gew.-%, insbesondere 0,01 bis 0,49 Gew.-%, insbesondere 0,01 bis 0,05 Gew.-% Saccharose, (jeweils bezogen auf Gesamtgewicht der Trockensubstanz des Isomalts) vorhanden sind. Ein derartiges, hier auch als hochreines Isomalt bezeichnetes Isomalt ist vorzugsweise und in vorteilhafter Weise insbesondere durch einen besonders hohen Anteil der Isomalt-Hauptkomponenten 1,1-GPM und 1,6-GPS, einen geringen, aber vorhandenen Anteil an Saccharose, eine hohe Reinheit in Hinblick auf Nebenprodukte sowie durch einen geringen Hygroskopizitätsgrad, insbesondere einen geringen Sorbit- und Mannitgehalt, ausgezeichnet.

In einer bevorzugten Ausführungsform der Erfindung wird insbesondere die Bildung mindestens einer, vorzugsweise aller nachstehend genannten Substanzen oder Substanzklassen (Nebenprodukte) statistisch signifikant reduziert im Vergleich zu nicht-erfindungsgemäß erhaltenem Isomalt: Trisaccharidalkohole, nicht reduzierende Trisaccharide, Glucosylglycerine, Glucosyltetritole, Glucosylpentitole, weitere Glucosylglycitole, Desoxydisaccharidalkohole, Didesoxydisaccharidalkohole, Glycerin, Tetritole (zum Beispiel Erythrit oder Threit), Pentitole (zum Beispiel Ribit (Adonit), Arabit, Xylit oder Lyxit), Desoxyhexitole, Didesoxyhexitole, Sorbit, Mannit, Galaktit, Allit, Gulit, Idit, Altrit, oder Talit.

Die Lehre der vorliegenden Erfindung sieht in überraschender Weise also vor, ein besonders selektives, effizientes und schonendes Verfahren zur Herstellung von Isomalt aus einer Kohlenhydratmischung, enthaltend Saccharose und Isomaltulose oder in einer Ausführungsform Saccharose, Isomaltulose und Trehalulose, bereitzustellen, in dem die Saccharose unter sauren Bedingungen während einer Ruthenium-basierten Hydrierung entgegen der Erwartungen aus dem Stand der Technik, zum Beispiel der EP 2 361 255 B1, nicht spaltend hydriert wird, sondern chemisch unverändert erhalten bleibt und nicht gespalten wird. Es kommt daher nicht zur Bildung von Sorbit und Mannit durch Saccharosespaltung und Hydrierung. Die erfindungsgemäße Vorgehensweise sieht somit vor, die spaltende Hydrierung von Saccharose zu Sorbit und Mannit, insbesondere Sorbit, während der Umsetzung der Saccharose-haltigen Kohlenhydratmischung enthaltend Isomaltulose oder Isomaltulose und Trehalulose mit Wasserstoff an einem Rutheniumkatalysator zu Isomalt zu vermeiden und so ein Isomalt bereitzustellen, welches über hervorragende Eigenschaften zur weiteren Verarbeitung in beispielsweise Süßigkeiten, wie Hartkaramellen, Dragees, Schokolade, Kaugummis, Eis oder in Backwaren oder in Medikamenten verfügt. Die Erfinder haben in überraschender Weise festgestellt, dass die in geringen Mengen im Ausgangsmaterial, das heißt der Kohlenhydratmischung, vorhandene Saccharose unter den erfindungsgemäßen Bedingungen, das heißt insbesondere Druck, Temperatur, pH-Wert und Raumgeschwindigkeit, nicht gespalten wird. Die "Restsaccharose", die üblicherweise in der durch Glucosylmutase-katalysierte Umsetzung von Saccharose oder Saccharose-haltigen Ausgangsmischungen gewonnenen "isomerisierten Saccharose" enthalten ist, trägt vorteilhafterweise dadurch, dass sie nicht gespalten und nicht hydriert wird, erfindungsgemäß nicht zu einer Erhöhung des Sorbit- und Mannit-Gehalts im erhaltenen Isomalt bei, sodass gegebenenfalls auch höhere Glucose- und Fructosegehalte in der in Verfahrensschritt a) bereitgestellten Isomaltulose- und Saccharose-haltigen Kohlenydratmischung eingesetzt werden können.

Die erfindungsgemäße Verfahrensweise ist also auch technologisch vorteilhaft, insofern als dass eine wesentlich moderatere Temperatur für die Isomaltulosehydrierung benötigt wird, als für eine gleichzeitige spaltende Hydrierung von Saccharose, da für die Hydrierung der aus der Spaltung der Saccharose neben Fructose resultierende Glucose eine deutlich höhere Temperatur notwendig ist. Darüber hinaus werden aufwendige und kostspielige Trocknungsschritte reduziert oder vermieden, die aus der Hygroskopizität von aus der spaltenden Hydrierung von Saccharose gebildetem Sorbit und/oder anderen Nebenprodukten resultieren würden und somit ein einfaches, prozessstabiles und kostenbewusstes Herstellverfahren für Isomalt, insbesondere ein 1,6-GPS angereichertes Isomalt, ermöglicht. Der geringe Anteil an Saccharose im hergestellten Isomalt ist vorteilhaft insofern, als dass das hergestellte Isomalt im Vergleich zu reiner Saccharose Diabetiker-geeignet, zahnschonend und brennwertreduziert ist. Das erfindungsgemäß hergestellte Süßungsmittel zeichnet sich insbesondere durch eine erhöhte Lagerstabilität, insbesondere in feuchten und heißen Klimaregionen, aus.

Im Zusammenhang mit der vorliegenden Erfindung wird unter "Inkontaktbringen" verstanden, dass das wässrige Medium, insbesondere eine wässrige Lösung, unter Wasserstoffzufuhr mit einem Katalysator und Wasserstoff in physischen Kontakt gebracht wird, insbesondere dass das Medium, insbesondere die Lösung, an dem Katalysator vorbei strömt, insbesondere durch ein den Katalysator enthaltendes Katalysatorbett strömt. Ohne an die Theorie gebunden zu sein, beschleunigt der Katalysator die Umsetzung der Isomaltulose oder der Isomaltulose und Trehalulose in der Kohlenhydratmischung, enthaltend Isomaltulose oder Isomaltulose und Trehalulose sowie Saccharose, mit Wasserstoff.

Das Inkontaktbringen der Kohlenhydratmischung mit dem Ruthenium-basierten Katalysator unter Wasserstoffzufuhr führt zu einer Umsetzung der Kohlenhydratmischung zu Isomalt. Erfindungsgemäß ist vorgesehen, dass das Inkontaktbringen der Kohlenhydratmischung mit dem Ruthenium-basierten Katalysator und Wasserstoff eine Hydrierung der Kohlenhydratmischung darstellt.

Im Zusammenhang mit der vorliegenden Erfindung wird unter dem Begriff der "Umsetzung einer Kohlenhydratmischung zu Isomalt" verstanden, dass die in der Kohlenhydratmischung vorhandene Isomaltulose teilweise oder vollständig mit Hilfe von Wasserstoff zu 1,6-GPS und 1,1-GPM umgesetzt, das heißt hydriert wird. Sofern in der Kohlenhydratmischung, enthaltend Isomaltulose und Saccharose, weitere hydrierfähige Bestandteile, beispielsweise Trehalulose, vorhanden sind, kann vorgesehen sein, dass auch diese während des Inkontaktbringens mit dem Ruthenium-basierten Katalysator umgesetzt, das heißt hydriert, werden, insbesondere Trehalulose zu 1,1-GPS und 1,1-GPM. Erfindungsgemäß findet während Verfahrensschritt b) eine Umsetzung der in der Kohlenhydratmischung, enthaltend Isomaltulose oder Isomaltulose und Trehalulose sowie Saccharose, vorhandenen Saccharose, insbesondere Restsaccharose, nicht statt. Eine Spaltung und/oder Hydrierung der Saccharose wird erfindungsgemäß vermieden.

Im Zusammenhang mit der vorliegenden Erfindung wird unter dem gemäß Verfahrensschritt b) erhaltenen "Reaktionsmedium" ein Medium verstanden, das sich bei kontinuierlichem Inkontaktbringen der im wässrigen Medium vorliegenden Kohlenhydratmischung unter Einfluss eines Ruthenium-basierten Katalysators und unter Wasserstoffzufuhr ausbildet, wobei dieses in bevorzugter Ausführungsform die in Verfahrensschritt a) bereitgestellten Komponenten Isomaltulose oder Isomaltulose und Trehalulose sowie Saccharose sowie die sich während der Umsetzung bildenden Produkte, insbesondere 1,6-GPS und 1,1-GPM, aufweist.

Im Zusammenhang mit der vorliegenden Erfindung wird unter dem Begriff "Raumgeschwindigkeit" der Quotient aus dem Volumenstrom der im wässrigen Medium vorliegenden Kohlenhydratmischung und dem Volumen des Ruthenium-basierten Katalysators Geschwindigkeit$_{Raum}$ = V (Flüssigkeitsvolumen)/V (Katalysatorvolumen) pro Stunde verstanden ($m^3/h \times m^3$ = 1/h, auch bezeichnet als LHSV: liquid hourly space velocity). Das Volumen des Katalysators bezieht sich auf das makroskopische Katalysatorvolumen, unabhängig von der Form beziehungsweise Struktur des Katalysators.

Im Zusammenhang mit der vorliegenden Erfindung wird unter "kontinuierlich" verstanden, dass die im wässrigen Medium vorliegende Kohlenhydratmischung mit einer Raumgeschwindigkeit, welche konstant größer 0 $h^{-1}$ ist, mit dem Katalysator in Kontakt gebracht wird.

Im Zusammenhang mit der vorliegenden Erfindung wird unter Isomalt ein Zuckeraustauschstoff verstanden, der als Hauptbestandteil 1,6-GPS und 1,1-GPM aufweist, insbesondere mindestens 86 Gew.-% 1,6-GPS und 1,1-GPM.

Ein "1,6-GPS-angereichertes Isomalt" ist ein Isomalt mit einem Anteil an 1,6-GPS, der größer als der 1,1-GPM-Anteil ist, das heißt einem 1,6-GPS- zu 1,1-GPM-Verhältnis von >1 (bezogen auf TS Gehalt an 1,6-GPS und 1,1-GPM in Isomalt).

In bevorzugter Ausführungsform wird unter dem Begriff Isomalt ein JECFA-gemäßes Isomalt verstanden. In einer besonders bevorzugten Ausführungsform wird unter dem Begriff Isomalt ein hochreines Isomalt verstanden.

Im Zusammenhang mit der vorliegenden Erfindung wird unter einem "JECFA-gemäßen Isomalt" ein Isomalt verstanden, das mindestens 98 Gew.-% hydrierte Mono- und Disaccharide, nämlich 1,6-GPS, 1,1-GPM, 1,1-GPS, Sorbit und Mannit, und höchstens 2 Gew.-% Nebenkomponenten aufweist, wobei in dem Isomalt mindestens 86 Gew.-% 1,6-GPS und 1,1-GPM, 0 bis 0,3 Gew.-% reduzierende Zucker und höchstens 0,50 Gew.-%, insbesondere 0,01 bis 0,50 Gew.-% Saccharose (jeweils bezogen auf Gesamtgewicht der Trockensubstanz des Isomalts) vorhanden sind.

Im Zusammenhang mit der vorliegenden Erfindung wird unter einem "hochreinen Isomalt" ein Isomalt verstanden, das mindestens 98 Gew.-% hydrierte Mono- und Disaccharide, nämlich 1,6-GPS, 1,1-GPM, 1,1-GPS, Sorbit und Mannit, und höchstens 2,00 Gew.-% Nebenkomponenten aufweist, wobei in dem Isomalt mindestens 98,00 Gew.-% 1,6-GPS und 1,1-GPM, 0 bis 0,50 Gew.-% Sorbit, 0 bis 0,50 Gew.-% Mannit, 0 bis 0,30 Gew.-% reduzierende Zucker, einzelne der vorgenannten 2 Gew.-% Nebenkomponenten, sofern vorhanden, in einer Menge von jeweils 0 bis 0,50 Gew.-% und insgesamt höchstens 0,50 Gew.-% reduzierende und nicht-reduzierende Zucker sowie höchstens 0,50 Gew.-%, insbesondere 0,01 bis 0,50 Gew.-% Saccharose (jeweils bezogen auf Gesamtgewicht der Trockensubstanz des Isomalts) vorhanden sind.

Im Zusammenhang mit der vorliegenden Erfindung wird unter dem Begriff "mindestens 98,00 Gew.-% hydrierte Mono- und Disaccharide" die mindestens 98,00 Gew.-% erfassende Menge an hydrierten Mono- und Disacchariden in der Isomalt-Zusammensetzung verstanden, die ausgewählt sind aus der Gruppe bestehend aus 1,6-GPS, 1,1-GPM, 1,1-GPS, Mannit und Sorbit.

Im Zusammenhang mit der vorliegenden Erfindung werden unter dem Begriff "höchstens 2,0 Gew.-% Nebenkomponenten" alle in einer Isomalt-Zusammensetzung vorhandenen Stoffe verstanden, die keine hydrierten Mono- oder Disaccharide ausgewählt aus der Gruppe bestehend aus 1,6-GPS, 1,1-GPM, 1,1-GPS, Mannit und Sorbit sind.

Im Zusammenhang mit der vorliegenden Erfindung sind "einzelne Nebenkomponente" individuelle Stoffe, die in ihrer Gesamtheit die Nebenkomponenten darstellen und wobei diese individuellen Stoffe, zum Beispiel Isomaltose, Saccharose, Glucose, Fructose, Isomaltulose, Glycerin, Glucopyranosylidit, Isomelezitose, jeweils einzelne in die Stoffgruppen der Monosaccharide, Disaccharide, Desoxydisaccharidalkohole, Trisaccharide, Glucosylglycerine, Glucosyltetritole, Glucosylpentitole, Trisaccharidalkohole, glucosylierte Disaccharidalkohole oder hydrierten Oligomere gehörende Stoffe sind.

Die erfindungsgemäß vorgesehenen und ermittelten Gehalte an Isomaltulose, Trehalulose und/oder Saccharose in der Kohlenhydratmischung sowie die Gehalte an 1,6-GPS (6-O-$\alpha$-D-Glucopyranosyl-D-sorbit), 1,1-GPM (1-O-$\alpha$-D-Glucopyranosyl-D-mannit), Saccharose, 1,1-GPS ($\alpha$-D-Glucopyranosyl-1,1-D-sorbit) und/oder Isomaltulose sowie gegebenenfalls von weiteren vorhandenen Komponenten im Isomalt werden bevorzugt mittels GC-FID (GC-Flammenionisationsdetektor) oder GC-Massenspektrometrie, besonders bevorzugt mittels GC-FID mit einer Bestimmungsgrenze von 0,01 g/100 g TS bei einem Signal/Rausch-Verhältnis von mindestens 10:1 gemäß FCC General Information/-Validation, United States Pharmacopeia und JECFA (1996), FNP52, Add/4 (Joint FAO/WHO Expert Committee on Food Additives), ermittelt.

Im Zusammenhang mit der vorliegenden Erfindung addieren, soweit nicht anders angegeben und/oder erkenntlich, die für eine Zusammensetzung von Komponenten angegebenen prozentualen Anteile einzelner Komponenten im Rahmen der jeweils angegebenen Prozentbereiche auf 100 Gew.-% der Zusammensetzung auf.

Sofern im Zusammenhang mit der vorliegenden Erfindung in einer Zahl die erste und zweite Nachkommastelle oder die zweite Nachkommastelle nicht angegeben sind/ist, sind/ist diese als 0 zu setzen.

Sofern im Zusammenhang mit der vorliegenden Erfindung ein "Vorhandensein" ein "Enthalten" oder ein "Aufweisen" einer Komponente in einer Menge von 0 Gew.-% erwähnt wird, bedeutet dies, dass die jeweilige Komponente nicht in messbarer Menge vorhanden, insbesondere nicht vorhanden ist.

Wenn nicht anders angegeben, wird im Zusammenhang mit der vorliegenden Erfindung unter dem Begriff "Kohlenhydratmischung" die Kohlenhydratmischung gemäß Verfahrensschritt a) verstanden, also ein Gemisch enthaltend Isomaltulose und Saccharose oder enthaltend Isomaltulose, Trehalulose und Saccharose.

Die erfindungsgemäß eingesetzte Kohlenhydratmischung umfasst Isomaltulose und Saccharose, insbesondere besteht aus diesen.

In einer bevorzugten Ausführungsform umfasst die Kohlenhydratmischung Isomaltulose, Trehalulose und Saccharose, insbesondere besteht aus diesen.

In einer besonders bevorzugten Ausführungsform umfasst die Kohlenhydratmischung Isomaltulose und Saccharose sowie mindestens eine weitere Substanz, insbesondere ausgewählt aus der Gruppe bestehend aus Fructose, Glucose, Isomaltose, Trehalulose und Oligomeren von Kohlenhydraten, insbesondere besteht aus diesen. In einer besonders bevorzugten Ausführungsform umfasst die Kohlenhydratmischung Isomaltulose, Trehalulose und Saccharose sowie mindestens eine weitere Substanz, insbesondere ausgewählt aus der Gruppe bestehend aus Fructose, Glucose, Isomaltose und Oligomeren von Kohlenhydraten, insbesondere besteht aus diesen.

Im Zusammenhang mit der vorliegenden Erfindung werden unter "Oligomeren von Kohlenhydraten" Oligomere und/oder Polymere von Monosacchariden mit mindestens drei Monosaccharideinheiten 3 und mit homogener oder heterogener Monosaccharid-Zusammensetzung verstanden.

Bevorzugt umfasst die Kohlenhydratmischung Isomaltulose und Saccharose sowie eine Substanz ausgewählt aus der Gruppe bestehend aus Trehalulose und Isomaltose, insbesondere besteht aus diesen. In einer besonders bevorzugten Ausführungsform der vorliegenden Erfindung weist die Isomaltulose- und Saccharose-haltige Kohlenhydratmischung neben Saccharose und Isomaltulose oder neben Saccharose, Isomaltulose und Trehalulose zusätzlich Glucose, Fructose, und Isomaltose auf, insbesondere besteht aus diesen, optional gemeinsam mit Oligomeren von Kohlenhydraten.

In einer besonders bevorzugten Ausführungsform der vorliegenden Erfindung weist die Kohlenhydratmischung jeweils höchstens 0,50 Gew.-%, insbesondere jeweils höchstens 0,40 Gew.-%, insbesondere höchstens 0,30 Gew.-%, insbesondere höchstens 0,20 Gew.-% (jeweils TS basierend auf Gesamt-Trockensubstanz der Kohlenhydratmischung) an Glucose, Fructose, Oligomeren von Kohlenhydraten und/oder Isomaltose auf.

In einer besonders bevorzugten Ausführungsform der vorliegenden Erfindung weist die Kohlenhydratmischung höchstens 2,00 Gew.-%, insbesondere höchstens 1,00 Gew.-%, insbesondere höchstens 0,50 Gew.-%, insbesondere höchstens 0,40 Gew.-% (jeweils TS basierend auf Gesamt-Trockensubstanz der Kohlenhydratmischung) einer Gesamtmenge an Glucose, Fructose, Oligomeren von Kohlenhydraten und/oder Isomaltose auf.

In einer besonders bevorzugten Ausführungsform der vorliegenden Erfindung weist die Kohlenhydratmischung höchstens 0,50 Gew.-%, insbesondere höchstens 0,40 Gew.-%, insbesondere höchstens 0,30 Gew.-%, insbesondere höchstens 0,20 Gew.-%, insbesondere höchstens 0,10 Gew.-% (jeweils TS basierend auf Gesamt-Trockensubstanz der Kohlenhydratmischung) Glucose auf.

In einer besonders bevorzugten Ausführungsform der vorliegenden Erfindung weist die Kohlenhydratmischung höchstens 0,50 Gew.-%, insbesondere höchstens 0,40 Gew.-%, insbesondere höchstens 0,30 Gew.-%, insbesondere höchstens 0,20 Gew.-%, insbesondere höchstens 0,10 Gew.-% (jeweils TS basierend auf Gesamt-Trockensubstanz der Kohlenhydratmischung) Fructose auf.

In einer besonders bevorzugten Ausführungsform der vorliegenden Erfindung weist die Kohlenhydratmischung höchstens 0,50 Gew.-%, insbesondere höchstens 0,40 Gew.-%, insbesondere höchstens 0,30 Gew.-%, insbesondere höchstens 0,20 Gew.-%, insbesondere höchstens 0,10 Gew.-% (jeweils TS basierend auf Gesamt-Trockensubstanz der Kohlenhydratmischung) Isomaltose auf.

In einer besonders bevorzugten Ausführungsform der vorliegenden Erfindung weist die Kohlenhydratmischung höchstens 0,50 Gew.-%, insbesondere höchstens 0,40 Gew.-%, insbesondere höchstens 0,30 Gew.-%, insbesondere höchstens 0,20 Gew.-%, insbesondere höchstens 0,10 Gew.-% (jeweils TS basierend auf Gesamt-Trockensubstanz der Kohlenhydratmischung) Oligomere von Kohlenhydraten auf.

In einer besonders bevorzugten Ausführungsform der vorliegenden Erfindung weist die in Verfahrensschritt a) bereitgestellte Kohlenhydratmischung keine Glucose oder keine Fructose oder keine Isomaltose, insbesondere keine Glucose und keine Fructose und keine Isomaltose, insbesondere keine Glucose und keine Fructose, insbesondere keine Glucose, insbesondere keine Fructose, auf.

In besonders bevorzugter Ausführungsform weist die in Verfahrensschritt a) bereitgestellte Kohlenhydratmischung keine Oligomeren von Kohlenhydraten auf.

In einer besonders bevorzugten Ausführungsform der vorliegenden Erfindung ist die in Verfahrensschritt a) bereitgestellte Kohlenhydratmischung, enthaltend Isomaltulose oder Isomaltulose und Trehalulose sowie Saccharose, eine Kohlenhydratmischung gewonnen durch die Umsetzung von Saccharose oder Saccharose-haltigen Ausgangsmischungen, insbesondere in wässrigem Medium, insbesondere wässriger Lösung, mit Saccharose-Glucosylmutasen.

In besonders bevorzugter Ausführungsform der vorliegenden Erfindung ist die in Verfahrensschritt a) bereitgestellte Kohlenhydratmischung eine sogenannte "isomerisierte Saccharose", insbesondere eine solche, die enzymatisch aus Saccharose oder Saccharose-haltigen Ausgangsmischungen hergestellt wurde, insbesondere so wie es in der EP 0 625 578 A1 beschrieben ist.

Hinsichtlich der Herstellung der "isomerisierten Saccharose" und der Mittel zu ihrer Herstellung wird der Offenbarungsgehalt in der genannten Patentanmeldung vollständig in den Offenbarungsgehalt der vorliegenden Lehre mit einbezogen.

Die erfindungsgemäß bevorzugt vorgesehene enzymatische Umsetzung von Saccharose oder Saccharose-haltigen Gemischen zu der gemäß Verfahrensschritt a) bereitgestellten Kohlenhydratmischung ist vorzugweise eine enzymatische Umsetzung mittels einer Saccharose-Glucosylmutase. In bevorzugter Weise kann die Umsetzung mittels einer Saccharose-Glucosylmutase durch Einsatz von Saccharose-Glucosylmutase aufweisenden Bakterien erfolgen, insbesondere ausgewählt aus der Gruppe bestehend aus Protaminobacter rubrum, Serratia plymuthica, Serratia marcescens, Erwinia rhapontici, Leuconostoc mesenteroides, Pseudomonos mesoacidophila, Agrobacterium radiobacter und Kombinationen davon.

In bevorzugter Ausführungsform betrifft die Erfindung daher ein Verfahren, wobei die in Verfahrensschritt a) bereitgestellte Isomaltulose- und Saccharose-haltige Kohlenhydratmischung durch enzymatische Umsetzung von Saccharose oder einer Saccharose-haltigen Ausgangsmischung mit einer Saccharose-Glucosylmutase erhalten wurde.

Die, vorzugsweise mittels Saccharose-Glucosylmutasen aus Saccharose oder Saccharose-haltiger Ausgangsmischung gewonnene, Kohlenhydratmischung kann entweder direkt gemäß Verfahrensschritt a) bereitgestellt und unmittelbar anschließend gemäß Verfahrensschritt b) umgesetzt werden oder es kann vor der Bereitstellung gemäß Verfahrensschritt a), in einer besonders bevorzugten Ausführungsform, ein Verfahrensschritt a0) zur Reduzierung des Saccharosegehaltes erfolgen. Der in Verfahrensschritt a0) optional vorgesehene Verfahrensschritt zur Reduzierung des Saccharosegehaltes ist insbesondere dann notwendig, wenn die in Verfahrensschritt a) bereitgestellte Kohlenhydratmischung aus einer Quelle, zum Beispiel einer durch enzymatische Umsetzung von Saccharose oder einer Saccharose-haltigen Ausgangsmischung mit einer Saccharose-Glucosylmutase gewonnen "isomerisierten Saccharose", gewonnen werden soll, in der ein höherer Saccharosegehalt vorliegt und demgemäß der Saccharosegehalt soweit reduziert werden muss, dass eine Kohlenhydratmischung mit dem Saccharosegehalt gemäß Verfahrensschritt a) erhalten wird.

In einer besonders bevorzugten Ausführungsform der vorliegenden Erfindung ist die in Verfahrensschritt a) bereitgestellte Kohlenhydratmischung eine durch enzymatische Umsetzung von Saccharose oder einer Saccharose-haltigen Ausgangsmischung mit einer Saccharose-Glucosylmutase erhaltene Kohlenhydratmischung und wird einem Verfahrensschritt a0) zur Reduzierung des Saccharosegehaltes auf einen Gehalt von 0,01 bis 0,50 Gew.-% Saccharose (Trockensubstanz bezogen auf Gesamt-Trockensubstanz der Kohlenhydratmischung) unterzogen.

In einer besonders bevorzugten Ausführungsform der vorliegenden Erfindung ist der Verfahrensschritt a0) eine Kristallisation zur teilweisen Abtrennung von Saccharose, eine Chromatographie an Ionenaustauschern zur teilweisen Abtrennung von Saccharose, eine enzymatische Spaltung von Saccharose, z.B. mittels Invertase, unter Beibehaltung einer Restsaccharosemenge von 0,01 bis 0,5 Gew.-% oder eine Kombination dieser Verfahren.

In einer besonders bevorzugten Ausführungsform der vorliegenden Erfindung enthält die Kohlenhydratmischung, bereitgestellt in Verfahrensschritt a), 0,01 bis 0,45 Gew.-%, insbesondere 0,01 bis 0,40 Gew.-%, insbesondere 0,01 bis 0,35 Gew.-%, insbesondere 0,01 bis 0,30 Gew.-%, insbesondere 0,01 bis 0,25 Gew.-%, insbesondere 0,01 bis 0,20 Gew.-%, insbesondere 0,01 bis 0,15 Gew.-%, insbesondere 0,01 bis 0,10 Gew.-%, insbesondere 0,01 bis 0,09 Gew.-%, insbesondere 0,01 bis 0,08 Gew.-%, insbesondere 0,01 bis 0,07 Gew.-%, insbesondere 0,01 bis 0,06 Gew.-%, insbesondere 0,01 bis 0,05 Gew.-%, insbesondere 0,01 bis 0,04 Gew.-%, insbesondere 0,01 bis 0,03 Gew.-%, insbesondere 0,01 bis 0,02 Gew.-%, insbesondere 0,02 bis 0,50 Gew.-%, insbesondere 0,02 bis 0,45 Gew.-%, insbesondere 0,02 bis 0,40 Gew.-%, insbesondere 0,02 bis 0,30 Gew.-%, insbesondere 0,02 bis 0,20 Gew.-%, insbesondere 0,02 bis 0,10 Gew.-%, insbesondere 0,02 bis 0,08 Gew.-%, insbesondere 0,02 bis 0,06 Gew.-%, insbesondere 0,02 bis 0,05 Gew.-%, insbesondere 0,02 bis 0,04 Gew.-% Saccharose (jeweils bezogen auf Trockensubstanz der Kohlenhydratmischung).

Vorzugsweise beträgt der Saccharosegehalt in der in Verfahrensschritt a) bereitgestellten Kohlenhydratmischung 0,01 bis 0,05 Gew.-% (bezogen auf Trockensubstanz der Kohlenhydratmischung).

In einer besonders bevorzugten Ausführungsform der vorliegenden Erfindung enthält die Kohlenhydratmischung, bereitgestellt in Verfahrensschritt a), 76,00 bis 99,99 Gew.-% Isomaltulose, insbesondere 78,00 bis 99,99 Gew.-%, insbesondere 80,00 bis 99,99 Gew.-%, insbesondere 84 bis 99,99 Gew.-%, insbesondere 86,00 bis 99,99 Gew.-%, insbesondere 90,00 bis 99,99 Gew.-%, insbesondere 92,00 bis 99,99 Gew.-%, insbesondere 94,00 bis 99,99 Gew.-%, insbesondere 96,00 bis 99,99 Gew.-%, insbesondere 98,00 bis 99,99 Gew.-%, insbesondere 76,00 bis 99,80 Gew.-%, insbesondere 78,00 bis 99,80 Gew.-%, insbesondere 80,00 bis 99,80 Gew.-%, insbesondere 84,00 bis 99,80 Gew.-%, insbesondere 86,00 bis 99,80 Gew.-%, insbesondere 90,00 bis 99,80 Gew.-%, insbesondere 92,00 bis 99,80 Gew.-%, insbesondere 94,00 bis 99,80 Gew.-%, insbesondere 96,00 bis 99,80 Gew.-%, insbesondere 98,00 bis 99,80 Gew.-%, insbesondere 76,00 bis 99,50 Gew.-%, insbesondere 78,00 bis 99,50 Gew.-%, insbesondere 80,00 bis 99,50 Gew.-%, insbesondere 84,00 bis 99,50 Gew.-%, insbesondere 86 bis 99,50 Gew.-%, insbesondere 90,00 bis 99,50 Gew.-%, insbesondere 92,00 bis 99,50 Gew.-%, insbesondere 94,00 bis 99,50 Gew.-%, insbesondere 95,00 bis 99,50 Gew.-%, insbesondere 96,00 bis 99,50 Gew.-%, insbesondere 97,00 bis 99,50 Gew.-%, insbesondere 98,00 bis 99,50 Gew.-%, insbesondere 97,70 bis 99,30 Gew.-% Isomaltulose (jeweils bezogen auf Trockensubstanz der Kohlenhydratmischung).

In einer besonders bevorzugten Ausführungsform weist die in Verfahrensschritt a) bereitgestellte Kohlenhydratmischung vorzugsweise einen Isomaltulosegehalt von 86,00 bis 99,99 Gew.-%, insbesondere 90,00 bis 99,99 Gew.-%, insbesondere 95,00 bis 99,99 Gew.-%, insbesondere 96,00 bis 99,99 Gew.-%, insbesondere 97,00 bis 99,99 Gew.-%, insbesondere 98,00 bis 99,99 Gew.-%, insbesondere 98,50 bis 99,99 Gew.-%, insbesondere 98,60 bis 99,99 Gew.-% (jeweils bezogen auf Trockensubstanz der Kohlenhydratmischung) auf.

Vorzugsweise beträgt der Isomaltulosegehalt der in Verfahrensschritt a) bereitgestellten Kohlenhydratmischung 86,00 bis 99,99 Gew.-% Isomaltulose (bezogen auf Trockensubstanz der Kohlenhydratmischung).

Vorzugsweise beträgt der Isomaltulosegehalt in der in Verfahrensschritt a) bereitgestellten Kohlenhydratmischung 98,00 bis 99,80 Gew.-% Isomaltulose (bezogen auf Trockensubstanz der Kohlenhydratmischung).

In einer besonders bevorzugten Ausführungsform der vorliegenden Erfindung weist die Kohlenhydratmischung Trehalulose auf.

In einer besonders bevorzugten Ausführungsform der vorliegenden Erfindung enthält die in Verfahrensschritt a) bereitgestellte, 75,00 bis 99,99 Gew.-% Isomaltulose und 0,01 bis 0.50 Gew.-% Saccharose-haltige Kohlenhydratmischung, 75,01 bis 100,00 Gew.-%, insbesondere 80,00 bis 95,00 Gew.-%, insbesondere 86,00 bis 90,00 Gew.-%, insbesondere 90,00 bis 99,00 Gew.-%, insbesondere 99,00 bis 100,00 Gew.-%, insbesondere 75,01 bis 99,99 Gew.-%, insbesondere 80,00 bis 99,99 Gew.-%, insbesondere 90,00 bis 99,99 Gew.-%, insbesondere 92,00 bis 99,99 Gew.-%, insbesondere 94,00 bis 99,99 Gew.-%, insbesondere 96,00 bis 99,99 Gew.-%, insbesondere 98,00 bis 99,99 Gew.-%, insbesondere 99,00 bis 99,99 Gew.-%, insbesondere 75,01 bis 99,80 Gew.-%, insbesondere 80,00 bis 99,80 Gew.-%, insbesondere 85,00 bis 99,80 Gew.-%, insbesondere 90,00 bis 99,80 Gew.-%, insbesondere 98,00 bis 99,80 Gew.-%, insbesondere 98,50 bis 99,80 Gew.-%, insbesondere 98,60 bis 99,80 Gew.-%, insbesondere 98,70 bis 99,80 Gew.-%, insbesondere 98,80 bis 99,80 Gew.-%, insbesondere 98,90 bis 99,80 Gew.-%, insbesondere 99,00 bis 99,80 Gew.-%, insbesondere 99,10 bis 99,80 Gew.-% Isomaltulose und Saccharose, wobei optional, auf 100 Gew.-% der Kohlenhydratmischung aufaddierend, Trehalulose, Glucose, Fructose, Isomaltose und/oder Oligomere von Kohlenhydraten vorhanden sind (jeweils bezogen auf Trockensubstanz der Kohlenhydratmischung).

Vorzugsweise beträgt der Saccharose- und Isomaltulose-Gehalt in der in Verfahrensschritt a) bereitgestellten Kohlenhydratmischung 98,00 bis 99,99 Gew.-% , wobei optional, auf 100 Gew.- % der Kohlenhydratmischung aufaddierend, Trehalulose, Glucose, Fructose, Isomaltose und/oder Oligomere von Kohlenhydraten vorhanden sind (bezogen auf Trockensubstanz der Kohlenhydratmischung).

Vorzugsweise beträgt der Saccharose-, Isomaltulose- und Trehalulose-Gehalt in der in Verfahrensschritt a) bereitgestellten Kohlenhydratmischung 98,00 bis 99,80 Gew.-% (bezogen auf Trockensubstanz der Kohlenhydratmischung), wobei der Isomaltulosegehalt 97,70 bis 99,30 Gew.-%, der Trehalulosegehalt 0,29 bis 1,00 Gew.-% und der Saccharosegehalt 0,01 bis 0,05 Gew.-% beträgt (jeweils bezogen auf Trockensubstanz der Kohlenhydratmischung).

Vorzugsweise beträgt der Saccharose-, Isomaltulose- und Trehalulose-Gehalt in der in Verfahrensschritt a) bereitgestellten Kohlenhydratmischung 98,00 bis 99,80 Gew.-% (bezogen auf Trockensubstanz der Kohlenhydratmischung), wobei der Isomaltulosegehalt 97,70 bis 99,30 Gew.-%, der Trehalulosegehalt 0,29 bis 1,00 Gew.-% und der Saccharosegehalt 0,01 bis 0,05 Gew.-% beträgt (jeweils bezogen auf Trockensubstanz der Kohlenhydratmischung), und wobei auf 100 Gew.-% der Kohlenhydratmischung aufaddierend Glucose, Fructose, Isomaltose und/oder Oligomere von Kohlenhydraten vorhanden sind (bezogen auf Trockensubstanz der Kohlenhydratmischung).

Vorzugsweise beträgt der Saccharose-, Isomaltulose- und Trehalulose-Gehalt in der in Verfahrensschritt a) bereitgestellten Kohlenhydratmischung 98 bis 99,80 Gew.-% (bezogen auf Trockensubstanz der Kohlenhydratmischung), wobei der Isomaltulose-Gehalt 98,00 bis 99,30 Gew.-%, der Trehalulose-Gehalt 0,29 bis 1,0 Gew.-% und der Saccharose-Gehalt 0,01 bis 0,05 Gew.-% beträgt (jeweils bezogen auf Trockensubstanz der Kohlenhydratmischung).

Vorzugsweise beträgt der Saccharose-, Isomaltulose- und Trehalulose-Gehalt in der in Verfahrensschritt a) bereitgestellten Kohlenhydratmischung 98 bis 99,80 Gew.-% (bezogen auf Trockensubstanz der Kohlenhydratmischung), wobei der Isomaltulose-Gehalt 98,00 bis 99,30 Gew.-%, der Trehalulose-Gehalt 0,29 bis 1,0 Gew.-% und der Saccharose-Gehalt 0,01 bis 0,05 Gew.-% beträgt (jeweils bezogen auf Trockensubstanz der Kohlenhydratmischung), und wobei auf 100 Gew.-% der Kohlenhydratmischung aufaddierend Glucose, Fructose, Isomaltose und/oder Oligomere von Kohlenhydraten vorhanden sind (bezogen auf Trockensubstanz der Kohlenhydratmischung).

In einer besonders bevorzugten Ausführungsform der vorliegenden Erfindung weist die in Verfahrensschritt a) bereitgestellte Isomaltulose- und Saccharose-haltige Kohlenhydratmischung 0,01 bis 24,99 Gew.-% Trehalulose, insbesondere 0,01 bis 24,50 Gew.-%, insbesondere 0,01 bis 21,00 Gew.-%, insbesondere 0,01 bis 20,00 Gew.-%, insbesondere 0,01 bis 19,00 Gew.-%, insbesondere 0,01 bis 18,00 Gew.-%, insbesondere 0,01 bis 17,00 Gew.-%, insbesondere 0,01 bis 10,00 Gew.-%, insbesondere 0,01 bis 5,00 Gew.-%, besonders bevorzugt 0,50 bis 25,00 Gew.-% Trehalulose, insbesondere 0,50 bis 23,00 Gew.-%, insbesondere 0,50 bis 21,00 Gew.-%, insbesondere 0,50 bis 20,00 Gew.-%, insbesondere 0,50 bis 19,00 Gew.-%, insbesondere 0,50 bis 18,00 Gew.-%, insbesondere 0,50 bis 17,00 Gew.-%, insbesondere 0,50 bis 10,00 Gew.-%, insbesondere 0,50 bis 5,00 Gew.-%, besonders bevorzugt 0,30 bis 1,00 Gew.-%, insbesondere bevorzugt 0,29 bis 1,00 Gew.-%, besonders bevorzugt 1,00 bis 25,00 Gew.-%, insbesondere 1,00 bis 23,00 Gew.-%, insbesondere 1,00 bis 21,00 Gew.-%, insbesondere 1,00 bis 20,00 Gew.-%, insbesondere 1,00 bis 10,00 Gew.-%, insbesondere 5,00 bis 25,00 Gew.-%, insbesondere 5,00 bis 23,00 Gew.-%, insbesondere 5,00 bis 22,00 Gew.-%, insbesondere 5,00 bis 20,00 Gew.-%, insbesondere 5,00 bis 10,00 Gew.-%, insbesondere 10,00 bis 25,00 Gew.-%, insbesondere 10,00 bis 22,00 Gew.-%, insbesondere 10,00 bis 20,00 Gew.-%, insbesondere 10,00 bis 18,00 Gew.-% Trehalulose auf (jeweils bezogen auf Trockensubstanz der Kohlenhydratmischung).

Bevorzugt weist die Kohlenhydratmischung 0,30 bis 1,00 Gew.-% Trehalulose auf (bezogen auf Trockensubstanz der Kohlenhydratmischung).

Sofern in einer weiteren besonders bevorzugten Ausführungsform die Kohlenhydratmischung neben Saccha-

**EP 3 849 991 B1**

rose und Isomaltulose auch Trehalulose aufweist, sind vorzugsweise in der Kohlenhydratmischung 75,00 bis 99,49 Gew.-%Isomaltulose und 0,50 bis 24,99 Gew.-% Trehalulose, insbesondere 75,00 bis 86,00 Gew.-% Isomaltulose und 13,99 bis 24,99 Gew.-% Trehalulose (jeweils bezogen auf Trockensubstanz der Kohlenhydratmischung) vorhanden.

In einer besonders bevorzugten Ausführungsform der vorliegenden Erfindung beträgt das Verhältnis von Isomaltulose zu Trehalulose in der Kohlenhydratmischung (Gew.-% in TS) 98,00 bis 99,50, insbesondere 98,50 bis 99,40 Teile Isomaltulose zu 0,50 bis 1,50, insbesondere 0,60 bis 1,20 Teile Trehalulose.

In bevorzugter Ausführungsform beträgt das Verhältnis von Isomaltulose zu Trehalulose (Gew.- % in TS) in der Kohlenhydratmischung 3 zu 1, insbesondere 4 zu 1, insbesondere 10 zu 1, insbesondere 85 zu 1, insbesondere 150 zu 1, insbesondere 1500 zu 1, insbesondere 9998 zu 1.

In besonders bevorzugter Ausführungsform ist das in Verfahrensschritt a) bereitgestellte wässrige Medium eine wässrige Lösung, eine wässrige Suspension, ein wässriger Sirup oder eine wässrige kolloidale Zusammensetzung.

In einer bevorzugten Ausführungsform der vorliegenden Erfindung beträgt der Trockensubstanzgehalt der in wässrigem Medium, insbesondere in wässriger Lösung, vorliegenden Kohlenhydratmischung 10,00 bis 60,00 Gew.-% (bezogen auf Gesamtgewicht des Mediums).

In einer besonders bevorzugten Ausführungsform der vorliegenden Erfindung beträgt der Trockensubstanzgehalt der in wässriger Lösung vorliegenden Kohlenhydratmischung 15,00 bis 60,00 Gew.-%, insbesondere 20,00 bis 60,00 Gew.-%, insbesondere 30,00 bis 60,00 Gew.-%, insbesondere 35,00 bis 60,00 Gew.-%, insbesondere 40,00 bis 60,00 Gew.-%, insbesondere 45,00 bis 60,00 Gew.-%, insbesondere 50,00 bis 60,00 Gew.-%, insbesondere 10,00 bis 55,00 Gew.-%, insbesondere 10,00 bis 50,00 Gew.-%, insbesondere 10,00 bis 45,00 Gew.-%, insbesondere 10,00 bis 40 Gew.-%, insbesondere 10,00 bis 35,00 Gew.-%, insbesondere 10,00 bis 30,00 Gew.-%, insbesondere 10,00 bis 25,00 Gew.-%, insbesondere 10,00 bis 20,00 Gew.-%, insbesondere 10,00 bis 15,00 Gew.-%, insbesondere 20,00 bis 50,00 Gew.-%, insbesondere 20,00 bis 45,00 Gew.-%, insbesondere 20,00 bis 30,00 Gew.-%, insbesondere 30,00 bis 50,00 Gew.-%, insbesondere 35,00 bis 50,00 Gew.-%, insbesondere 35,00 bis 45,00 Gew.-% (jeweils bezogen auf Gesamtgewicht des Mediums, insbesondere der Lösung).

Bevorzugt beträgt der Trockensubstanzgehalt der in wässriger Lösung vorliegenden Kohlenhydratmischung 35,00 bis 45,00 Gew.-%, bezogen auf Gesamtgewicht des Mediums.

In einer besonders bevorzugten Ausführungsform ist vorgesehen, dass die in Verfahrensschritt a) bereitgestellte Kohlenhydratmischung vor der im Verfahrensschritt b) vorgesehenen Umsetzung in einem Verfahrensschritt a1) erwärmt wird, das heißt vortemperiert wird.

In besonders bevorzugter Ausführungsform wird die in Verfahrensschritt a) bereitgestellte, insbesondere nach Durchführung eines optionalen den Saccharosegehalt reduzierenden Verfahrensschritts a0), Kohlenhydratmischung vor der in Verfahrensschritt b) vorgesehenen Umsetzung auf eine Temperatur vorgewärmt, die höchstens der Reaktionstemperatur gemäß Verfahrensschritt b) entspricht, insbesondere auf eine Temperatur, die der Reaktionstemperatur gemäß Verfahrensschritt b) entspricht.

In besonders bevorzugter Ausführungsform wird in einem Verfahrensschritt a1) die im Verfahrensschritt a) bereitgestellte, insbesondere nach Durchführung eines optionalen den Saccharosegehalt reduzierenden Verfahrensschritts a0), Isomaltulose- und Saccharose-haltige Kohlenhydratmischung auf eine Temperatur von 30 bis 80 °C, insbesondere 30 bis 75 °C, insbesondere 30 bis 70 °C, insbesondere 45 bis 68 °C, insbesondere 46 bis 67,5 °C vorgewärmt.

In einer besonders bevorzugten Ausführungsform wird in einem Verfahrensschritt a1) die im Verfahrensschritt a) bereitgestellt, insbesondere nach Durchführung eines optionalen den Saccharosegehalt reduzierenden Verfahrensschritts a0), Isomaltulose- und Saccharose-haltige Kohlenhydratmischung auf eine Temperatur von 30 bis 80 °C vorgewärmt.

In einer besonders bevorzugten Ausführungsform wird in einem Verfahrensschritt a1) die im Verfahrensschritt a) bereitgestellte, insbesondere nach Durchführung eines optionalen den Saccharosegehalt reduzierenden Verfahrensschritts a0), Isomaltulose- und Saccharose-haltige Kohlenhydratmischung auf eine Temperatur von 30 bis 75 °C vorgewärmt.

In einer besonders bevorzugten Ausführungsform wird in einem Verfahrensschritt a1) die im Verfahrensschritt a) bereitgestellte, insbesondere nach Durchführung eines optionalen den Saccharosegehalt reduzierenden Verfahrensschritts a0), Isomaltulose- und Saccharose-haltige Kohlenhydratmischung auf eine Temperatur von 30 bis 70 °C vorgewärmt.

Die in Verfahrensschritt a) bereitgestellte Kohlenhydratmischung oder die in optionaler Form gemäß Verfahrensschritt a1) vortemperierte Kohlenhydratmischung wird anschließend dem Verfahrensschritt b) zugeführt, insbesondere in einen für die in Verfahrensschritt b) vorgesehene Umsetzung geeigneten Reaktor eingespeist.

In bevorzugter Ausführungsform wird das gemäß Verfahrensschritt b) vorgesehene kontinuierliche Verfahren in einem Festbett-Reaktor durchgeführt.

In bevorzugter Ausführungsform wird das gemäß Verfahrensschritt b) vorgesehene kontinuierliche Verfahren als Trickle-bed- Verfahrensweise durchgeführt, vorzugsweise in einem Trickle-bed-Reaktor.

11

In bevorzugter Ausführungsform kann die kontinuierliche Verfahrensweise auch in einem Festbett-Reaktor durchgeführt werden, der als Blasenreaktor betrieben wird.

In bevorzugter Ausführungsform kann die kontinuierliche Verfahrensweise auch in einem kontinuierlichen Rührkessel (CSTR) oder in einer Rührkesselkaskade durchgeführt werden.

In einer besonders bevorzugten Ausführungsform der Erfindung beträgt der Wasserstoffdruck in Verfahrensschritt b) 16,2 bis 22,0 MPa, insbesondere 16,5 bis 22,0 MPa, insbesondere 16,5 bis 21,5 MPa, insbesondere 16,5 bis 21,0 MPa, insbesondere 16,5 bis 20,0 MPa, insbesondere 16,5 bis 19,5 MPa, insbesondere 16,5 bis 19,0 MPa, insbesondere 16,5 bis 18,5 MPa, insbesondere 16,5 bis 18,0 MPa.

In bevorzugter Ausführungsform beträgt der Wasserstoffdruck in Verfahrensschritt b) 17,0 bis 22,0 MPa, insbesondere 17,0 bis 21,0 MPa, insbesondere 17,0 bis 20,0 MPa, insbesondere 17,0 bis 19,5 MPa, insbesondere 17,0 bis 19,0 MPa, insbesondere 17,0 bis 18,5 MPa, insbesondere 17,5 bis 22,0 MPa, insbesondere 17,5 bis 19,0 MPa, insbesondere 17,5 bis 18,5 MPa, insbesondere 17,5 bis 18,0 MPa, insbesondere 18,0 MPa.

Bevorzugt beträgt der Wasserstoffdruck in Verfahrensschritt b) 16,5 bis 21,0 MPa.

In einer besonders bevorzugten Ausführungsform beträgt der Wasserstoffdruck in Verfahrensschritt b) 16,00 bis 19,00 MPa, insbesondere 16,00 bis 17,00 MPa, insbesondere 16,5 MPa.

In einer besonders bevorzugten Ausführungsform der vorliegenden Erfindung beträgt die Raumgeschwindigkeit in Verfahrensschritt b) 0,27 bis 1,5 $h^{-1}$, insbesondere 0,3 bis 1,5 $h^{-1}$, insbesondere 0,3 bis 1,0 $h^{-1}$, insbesondere 0,3 bis 0,9 $h^{-1}$, insbesondere 0,3 bis 0,8 $h^{-1}$, insbesondere 0,3 bis 0,7 $h^{-1}$, insbesondere 0,3 bis 0,6 $h^{-1}$, insbesondere 0,3 bis 0,5 $h^{-1}$, insbesondere 0,4 bis 1,5 $h^{-1}$, insbesondere 0,4 bis 1,25 $h^{-1}$, insbesondere 0,4 bis 1,0 $h^{-1}$, insbesondere 0,4 bis 0,8 $h^{-1}$, insbesondere 0,4 bis 0,7 $h^{-1}$, insbesondere 0,4 bis 0,6 $h^{-1}$, insbesondere 0,25 bis 1,0 $h^{-1}$, insbesondere 0,25 bis 0,9 $h^{-1}$, insbesondere 0,25 bis 0,8 $h^{-1}$, insbesondere 0,25 bis 0,7 $h^{-1}$, insbesondere 0,25 bis 0,6 $h^{-1}$, insbesondere 0,25 bis 0,5 $h^{-1}$, insbesondere 0,45 bis 1,5 $h^{-1}$, insbesondere 0,46 bis 1,5 $h^{-1}$, insbesondere 0,48 bis 1,5 $h^{-1}$, insbesondere 0,48 bis 1,0 $h^{-1}$, insbesondere genau 0,3 $h^{-1}$, insbesondere genau 0,4 $h^{-1}$, insbesondere genau 0,5 $h^{-1}$, insbesondere genau 0,7 $h^{-1}$, insbesondere genau 1,0 $h^{-1}$.

In einer besonders bevorzugten Ausführungsform der vorliegenden Erfindung beträgt die Raumgeschwindigkeit in Verfahrensschritt b) 0,25 bis 0,9 $h^{-1}$.

In einer besonders bevorzugten Ausführungsform der vorliegenden Erfindung beträgt die Raumgeschwindigkeit im Verfahrensschritt b) 0,3 bis 0,9 $h^{-1}$.

In einer besonders bevorzugten Ausführungsform der vorliegenden Erfindung beträgt der pH-Wert in Verfahrensschritt b) 2,0 bis 5,5, insbesondere 2,0 bis 5,4, insbesondere 2,0 bis 5,3, insbesondere 2,0 bis 5,0, insbesondere 2,0 bis 4,0, insbesondere 2,5 bis 6,0, insbesondere 2,5 bis 5,8, insbesondere 2,5 bis 5,5, insbesondere 2,5 bis 4,0, insbesondere 2,7 bis 3,3, insbesondere 2,8 bis 3,0, insbesondere 3,0 bis 6,0, insbesondere 3,0 bis 5,5, insbesondere 3,0 bis 5,0, insbesondere 3,0 bis 4,5, insbesondere 3,0 bis 4,0, insbesondere 3,0 bis 4,0, insbesondere 4,0 bis 6,0 insbesondere 4,0 bis 5,5, insbesondere 4,0 bis 5,0, insbesondere 4,0 bis 4,5, insbesondere 4,0 bis 4,0, insbesondere 4,0 bis 6,0, insbesondere 4,0 bis 5,5, insbesondere 4,0 bis 5,0, insbesondere 4,0 bis 4,5, insbesondere 4,5 bis 6,0, insbesondere 4,5 bis 5,5, insbesondere 4,5 bis 5,0, insbesondere 5,0 bis 6,0.

In einer besonders bevorzugten Ausführungsform beträgt der pH-Wert in Verfahrensschritt b) 2,5 bis 6,0, insbesondere 2,5 bis 5,8, insbesondere 2,5 bis 5,5.

In einer besonders bevorzugten Ausführungsform der vorliegenden Erfindung beträgt der pH-Wert in Verfahrensschritt b) 2,5 bis 5,9.

In einer besonders bevorzugten Ausführungsform der vorliegenden Erfindung beträgt der pH-Wert in Verfahrensschritt b) 3,4 bis 5,9.

In einer besonders bevorzugten Ausführungsform der vorliegenden Erfindung beträgt die Reaktionstemperatur höchstens 98 °C, insbesondere höchstens 95 °C, insbesondere höchstens 91 °C, insbesondere höchstens 85 °C, insbesondere höchstens 82 °C, insbesondere höchstens 79°C, insbesondere höchstens 78 °C, insbesondere höchstens 72 °C, insbesondere höchstens 70°C, insbesondere höchstens 65 °C, insbesondere höchstens 60 °C, insbesondere höchstens 55 °C.

In einer besonders bevorzugten Ausführungsform der vorliegenden Erfindung beträgt die Reaktionstemperatur in Verfahrensschritt b) 30 bis 100 °C, insbesondere 30 bis 98 °C, insbesondere 32 bis 95 °C, insbesondere 30 bis 91 °C, insbesondere 30 bis 79 °C, insbesondere 30 bis 75 °C, insbesondere 30 bis 70 °C, insbesondere 30 bis 60 °C, insbesondere 30 bis 50 °C, insbesondere 40 bis 100 °C, insbesondere 40 bis 98 °C, insbesondere 40 bis 95 °C, insbesondere 40 bis 91 °C, insbesondere 40 bis 79 °C, insbesondere 40 bis 75 °C, insbesondere 40 bis 70 °C, insbesondere 40 bis 60 °C, insbesondere 45 bis 100 °C, insbesondere 45 bis 98 °C, insbesondere 45 bis 95 °C, insbesondere 45 bis 91 °C, insbesondere 45 bis 79 °C, insbesondere 45 bis 75 °C insbesondere 45 bis 70 °C, insbesondere 45 bis 60 °C, insbesondere 50 bis 100 °C, insbesondere 50 bis 98 °C, insbesondere 50 bis 95 °C, insbesondere 50 bis 91 °C, insbesondere 50 bis 80 °C, insbesondere 50 bis 70 °C, insbesondere 50 bis 60 °C, insbesondere 55 bis 100 °C, insbesondere 55 bis 98 °C, insbesondere 55 bis 95 °C, insbesondere 55 bis 91 °C, insbesondere 55 bis 79 °C, insbesondere 55 bis 75 °C insbesondere 55 bis 70 °C, insbesondere 60 bis 100 °C, insbesondere 60 bis 98 °C, insbesondere 60 bis 95 °C, insbesondere 60 bis 91 °C, insbesondere 60 bis 79 °C, insbesondere 60 bis 75 °C insbesondere 60 bis 70 °C, insbesondere 65 bis 100 °C, insbesondere 65 bis 98 °C, insbesondere 65 bis 95 °C, insbesondere 65 bis 91 °C, insbesondere 65 bis 85 °C, insbesondere 65 bis 79 °C, insbesondere 68 bis 100 °C, insbesondere 68 bis 98 °C, insbesondere 68 bis 95 °C, insbesondere 68 bis 91 °C, insbesondere 68 bis 79 °C, insbesondere 70 bis 100 °C, insbesondere 70 bis 98 °C, insbesondere 70 bis 95 °C, insbesondere 70 bis 91 °C, insbesondere 70 bis 79 °C.

In einer besonders bevorzugten Ausführungsform der vorliegenden Erfindung beträgt die Reaktionstemperatur in Verfahrensschritt b) 70 bis 95 °C.

In einer besonders bevorzugten Ausführungsform wird in Verfahrensschritt a1) die Kohlenhydratmischung auf 30 bis 70 °C vortemperiert und in Verfahrensschritt b) eine Reaktionstemperatur von 70 bis 95 °C eingestellt.

In einer bevorzugten Ausführungsform der Erfindung wird die Isomaltulose in Verfahrensschritt b) mit einer Umsatzrate von 99,5 bis 100 mol-%, insbesondere 99,9 bis 100 mol-%, zu 1,6-GPS (6-O-$\alpha$-D-Glucopyranosyl-D-sorbit) und 1,1-GPM (1-O-$\alpha$-D-Glucopyranosyl-D-mannit) umgesetzt.

In einer bevorzugten Ausführungsform der Erfindung wird die Isomaltulose in Verfahrensschritt b) mit einer Selektivität von 98 bis 100 mol-%, insbesondere 99 bis 100 mol-%, insbesondere 99,5 bis 100 mol-%, zu 1,6-GPS (6-O-$\alpha$-D-Glucopyranosyl-D-sorbit) und 1,1-GPM (1-O-$\alpha$-D-Glucopyranosyl-D-mannit) umgesetzt.

In einer besonders bevorzugten Ausführungsform der Erfindung beträgt der Wasserstoffdruck 16,5 bis 21,0 MPa, die Reaktionstemperatur 30 bis 95 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 50 bis 95 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 80 bis 95 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 80 °C.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 21,0 MPa, die Reaktionstemperatur 30 bis 79 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 50 bis 79 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 70 bis 79 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 21,0 MPa, die Reaktionstemperatur 70 bis 79 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 18,0 MPa, die Reaktionstemperatur 30 bis 95 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 18,0 MPa, die Reaktionstemperatur 50 bis 95 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 18,0MPa, die Reaktionstemperatur 70 bis 95 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 80 °C.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 18,0 MPa, die Reaktionstemperatur 30 bis 79 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 18,0 MPa, die Reaktionstemperatur 50 bis 79 °C, die Raumgeschwindigkeit 0,3 bis 0,9 h$^{-1}$ und der pH-Wert 2,5 bis 6,0.

In einer besonders bevorzugten Ausführungsform der Erfindung beträgt der Wasserstoffdruck 16,5 bis 18,0 MPa, die Reaktionstemperatur 70 bis 79 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 18,0 MPa, die Reaktionstemperatur 70 bis 79 °C, die Raumgeschwindigkeit 0,3 bis 0,9 h$^{-1}$ und der pH-Wert 2,5 bis 6,0, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 21,0 MPa, die Reaktionstemperatur 30 bis 95 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 21,0 MPa, die Reaktionstemperatur 50 bis 95 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 80 °C.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 21,0 MPa, die Reaktionstemperatur 30 bis 79 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 21,0 MPa, die Reaktionstemperatur 50 bis 79 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 21,0 MPa, die Reaktionstemperatur 70 bis 79 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 21,0 MPa, die Reaktionstemperatur 70 bis 79 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 18,0 MPa, die Reaktionstemperatur 30 bis 95 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 18,0 MPa, die Reaktionstemperatur 50 bis 95 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 80 °C.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 18,0 MPa, die Reaktionstemperatur 30 bis 79 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 18,0 MPa, die Reaktionstemperatur 50 bis 79 °C, die Raumgeschwindigkeit 0,25 bis 0,9 h$^{-1}$ und der pH-Wert 3,4 bis 5,9.

In einer besonders bevorzugten Ausführungsform der Erfindung beträgt der Wasserstoffdruck 16,5 bis 18,0 MPa, die Reaktionstemperatur 70 bis 79 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 18,0 MPa, die Reaktionstemperatur 70 bis 79 °C, die Raumgeschwindigkeit 0,25 bis 0,9 h$^{-1}$ und der pH-Wert 3,4 bis 5,9, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 21,0 MPa, die Reaktionstemperatur 30 bis 95 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 50 bis 95 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 80 °C.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 21,0 MPa, die Reaktionstemperatur 70 bis 95 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 21,0 MPa, die Reaktionstemperatur 30 bis 79 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 50 bis 79 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 21,0 MPa, die Reaktionstemperatur 70 bis 79 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 21,0 MPa, die Reaktionstemperatur 70 bis 79 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 18,0 MPa, die Reaktionstemperatur 30 bis 95 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 18,0 MPa, die Reaktionstemperatur 50 bis 95 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 80 °C.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 18,0 MPa, die Reaktionstemperatur 70 bis 95 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 70 °C.

In einer besonders bevorzugten Ausführungsform der Erfindung beträgt der Wasserstoffdruck 16,5 bis 18,0 MPa, die Reaktionstemperatur 30 bis 79 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 18,0 MPa, die Reaktionstemperatur 50 bis 79 °C, die Raumgeschwindigkeit 0,25 bis 1,5 h$^{-1}$ und der pH-Wert 2,5 bis 6,0.

In einer besonders bevorzugten Ausführungsform der Erfindung beträgt der Wasserstoffdruck 16,5 bis 18,0 MPa, die Reaktionstemperatur 70 bis 79 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 75 °C.

In einer besonders bevorzugten Ausführungsform der Erfindung beträgt der Wasserstoffdruck 16,5 bis 18,0 MPa, die Reaktionstemperatur 70 bis 79 °C, die Raumgeschwindigkeit 0,25 bis 1,5 h$^{-1}$ und der pH-Wert 2,5 bis 6,0, insbesondere nach Vortemperierung in einem Verfahrensschritt a1) auf 30 bis 70 °C.

Im Zusammenhang mit der vorliegenden Erfindung wird unter einem "Ruthenium-basierten Katalysator" ein Katalysator verstanden, welcher elementares Ruthenium und/oder Rutheniumoxid und/oder eine Ruthenium-haltige Verbindung, zum Beispiel ein Rutheniumsalz, aufweist.

Insbesondere weist der Katalysator 0,05 bis 20,00 Gew.-%, insbesondere 0,05 bis 5,00 Gew.-%, 0,10 bis 20,00 Gew.-%, insbesondere 0,30 bis 10,00 Gew.-%, insbesondere 0,50 bis 5,00 Gew.- % Ruthenium (jeweils bezogen auf elementares Ruthenium und das Trockengewicht des Katalysators) auf.

In bevorzugter Ausführungsform kann das Rutheniumoxid Sesquioxid, Dioxid oder Tetraoxid sein.

In bevorzugter Ausführungsform kann das Rutheniumsalz Ruthenium-Nitrosylnitrat, Ruthenium-Acetylacetonat, Bariumperruthenit, Natriumperruthenit, ein Ruthenat wie Magnesium-, Strontium-, Calcium-, Silber-, Barium-, Kalium- oder Natriumruthenat, ein Perruthenat wie Natrium- oder Kaliumperruthenat, ein Rutheniumhalid wie Rutheniumdichlorid, Rutheniumtrichlorid, Rutheniumtetrachlorid, Rutheniumpentafluorid, ein Rutheniumsulfid wie Rutheniumdisulfid oder ein Chlorsalz von Ruthenium wie Kaliumchloroperruthenat sein.

Im Zusammenhang mit der vorliegenden Erfindung wird unter dem Begriff "Ruthenium", sofern nicht anders angegeben, elementares Ruthenium oder eine Ruthenium-haltige Verbindung verstanden.

Der Ruthenium-basierte Katalysator kann ein monometallischer Katalysator sein, insbesondere als katalytisch aktives Metall allein Ruthenium aufweisen, oder in einer weiteren Ausführungsform auch ein bimetallischer Katalysator sein, der neben Ruthenium ein weiteres Metall enthält, zum Beispiel Nickel, Palladium, Platin, Iridium, Kobalt, Rhenium, Osmium, Gold, Silber, oder Kupfer.

Sofern gemäß bevorzugter Ausführungsform ein bimetallischer Katalysator vorliegt, kann dieser in bevorzugter Ausführungsform 5,00 bis 95,00 Gew.-% Ruthenium, vorzugsweise mindestens 50,00 Gew.-%, insbesondere mindestens 55 Gew.-%, Ruthenium enthalten (jeweils bezogen auf elementare katalytisch aktive Metalle und Gesamtgewicht der katalytisch aktiven Metalle des Katalysators).

In einer besonders bevorzugten Ausführungsform der vorliegenden Erfindung ist der Ruthenium-basierte Katalysator ein auf einem Träger immobilisierter Katalysator.

In einer besonders bevorzugten Ausführungsform der vorliegenden Erfindung ist der Träger ein saurer Träger, insbesondere ein intrinsisch saurer Träger, also ein Träger, der aufgrund seiner chemischen Zusammensetzung sauer wirkt oder ein Träger, der durch Aufbringen von sauren Funktionen, sauer wirkt.

In einer besonders bevorzugten Ausführungsform der vorliegenden Erfindung ist dieser Träger Kohlenstoff, ein Metalloxid, insbesondere Aluminiumoxid ($Al_2O_3$), Titandioxid ($TiO_2$) oder Siliziumdioxid ($SiO_2$), Zirkoniumdioxid ($ZrO_2$), oder ein Zeolith, beispielsweise ein Zeolith vom H-Y-Typ.

In einer besonders bevorzugten Ausführungsform kann der erfindungsgemäß eingesetzte Katalysator in bekannter Weise, wie im Lehrbuch "Technische Katalyse - Eine Einführung" von Jens Hagen (VCH Weinheim, 1996) beschrieben, hergestellt werden, indem der Katalysatorträger zunächst mit einer Lösung eines Rutheniumsalzes imprägniert oder beschichtet, anschließend der so behandelte Träger getrocknet, erhitzt und einem reduzierenden Gasstrom ausgesetzt wird.

Im erfindungsgemäßen Verfahrensschritt b) wird die bereitgestellte Kohlenhydratmischung zu Isomalt umgesetzt und kann im darauffolgenden Verfahrensschritt c) aus dem wässrigen Reaktionsmedium erhalten, insbesondere isoliert, werden. Zu diesem Zweck können übliche Isolationsverfahren, beispielsweise Kristallisationsverfahren, eingesetzt werden.

In einer bevorzugten Ausführungsform kann das Isomalt in Verfahrensschritt c) in fester, trockener Form durch entsprechende Isolations-, z. B. Kristallisationsverfahren und Trocknungsverfahren, erhalten werden.

In bevorzugter Ausführungsform kann vorgesehen sein, das in flüssiger Form vorliegende Isomalt durch Verdampfer, Trockner, insbesondere Sprühtrockner, Fallfilmverdampfer, Trommeltrockner oder sonstige übliche Vorrichtungen zu trocknen.

In einer weiteren Ausführungsform kann in Verfahrensschritt c) das Isomalt in flüssiger, beispielsweise gelöster oder suspendierter Form erhalten werden, insbesondere durch Aufkonzentrationsschritte, beispielsweise Verdampfungsschritte oder Membranverfahren.

In bevorzugter Ausführungsform kann das in Verfahrensschritt c) erhaltende Isomalt in flüssiger, halbflüssiger oder trockener Form, insbesondere in kristalliner Form, vorliegen.

Die vorliegende Erfindung betrifft auch Isomalt, herstellbar nach einem der erfindungsgemäßen Verfahren, insbesondere erhalten in Verfahrensschritt c).

In bevorzugter Ausführungsform ist das erfindungsgemäß in Verfahrensschritt c) hergestellte Isomalt ein Zuckeraustauschstoff, der als Hauptbestandteile 1,6-GPS und 1,1-GPM aufweist, insbesondere mindestens 86,00 Gew.-% 1,6-GPS und 1,1-GPM (bezogen auf Gesamt-TS des Isomalts). In bevorzugter Ausführungsform ist dieses Isomalt ein 1,6-GPS- und 1,1-GPM-haltiges Gemisch mit einem 1,6-GPS- zu 1,1-GPM-Verhältnis von > 1, insbesondere 55 bis 62 Gew.-% 1,6-GPS und 38 bis 45 Gew.-% 1,1-GPM (bezogen auf TS der Gesamtmenge von 1,6-GPS und 1,1-GPM in Isomalt).

In besonderes bevorzugter Ausführungsform ist das erfindungsgemäß hergestellte Isomalt ein JECFA-gemäßes Isomalt, welches mindestens 98,00 Gew.-% hydrierte Mono- und Disaccharide, nämlich 1,6-GPS, 1,1-GPM, 1,1-GPS, Sorbit und Mannit, und höchstens 2,00 Gew.-% Nebenkomponenten aufweist, wobei in dem Isomalt mindestens 86 Gew.-% 1,6-GPS und 1,1-GPM, 0 bis 0,30 Gew.-% reduzierende Zucker und höchstens 0,50 Gew.-%, insbesondere 0,01 bis 0,50 Gew.-% Saccharose vorhanden sind (jeweils basierend auf Gesamt-TS des Isomalts).

Ein derartiges JECFA-gemäßes Isomalt ist in bevorzugter Ausführungsform ein 1,6-GPS- und 1,1-GPM-haltiges Gemisch mit einem 1,6-GPS- zu 1,1-GPM-Verhältnis von >1, insbesondere 55 bis 62 Gew.-% 1,6-GPS und 38 bis 45 Gew.-% 1,1-GPM (bezogen auf TS der Gesamtmenge von 1,6-GPS und 1,1-GPM in Isomalt).

Isomalt, insbesondere JECFA-gemäßes Isomalt kann in bevorzugter Ausführungsform aus einer in Verfahrensschritt a) bereitgestellten Isomaltulose- und Saccharose-haltigen Kohlenhydratmischung hergestellt werden, die 86,00 bis 99,99 Gew.-%, insbesondere 98,00 bis 99,99 Gew.-% Isomaltulose und höchstens 0,50 Gew.-%, insbesondere 0,01 bis 0,50 Gew.-% Saccharose aufweist (jeweils TS (Trockensubstanz), bezogen auf Gesamt-TS der Kohlenhydratmischung).

Isomalt, insbesondere JECFA-gemäßes Isomalt kann in bevorzugter Ausführungsform aus einer in Verfahrensschritt a) bereitgestellten Isomaltulose- und Saccharose-haltigen Kohlenhydratmischung hergestellt werden, die 86,00 bis 99,99 Gew.-%, insbesondere 98,00 bis 99,99 Gew.-% Isomaltulose und höchstens 0,50 Gew.-%, insbesondere 0,01 bis 0,50 Gew.-% Saccharose und optional, auf 100 Gew.-% der Kohlenhydratmischung (TS) aufaddierend, Trehalulose, Isomaltose, Glucose, Fructose und/oder Oligomere von Kohlenhydraten aufweist, wobei wenn Oligomere von Kohlenhydraten vorhanden sind, diese in einer Menge von höchstens 0,5 Gew.-% vorliegen (jeweils TS (Trockensubstanz), bezogen auf Gesamt-TS der Kohlenhydratmischung).

Isomalt, insbesondere ein JECFA-gemäßes Isomalt kann in bevorzugter Ausführungsform auch aus einer in Verfahrensschritt a) bereitgestellten Isomaltulose- und Saccharose-haltigen Kohlenhydratmischung hergestellt werden, die 75,00 bis 99,49 Gew.-% Isomaltulose, 0,50 bis 24,99 Gew.-% Trehalulose und höchstens 0,50 Gew.-%, insbesondere 0,01 bis 0,50 Gew.-% Saccharose (jeweils TS bezogen auf Gesamt-TS der Kohlenhydratmischung) aufweist.

Isomalt, insbesondere ein JECFA-gemäßes Isomalt kann in bevorzugter Ausführungsform auch aus einer in Verfahrensschritt a) bereitgestellten Isomaltulose- und Saccharose-haltigen Kohlenhydratmischung hergestellt werden, die 75,00 bis 99,49 Gew.-% Isomaltulose, 0,50 bis 24,99 Gew.-% Trehalulose und höchstens 0,50 Gew.-%, insbesondere 0,01 bis 0,50 Gew.-% Saccharose und, optional auf 100 Gew.-% der Kohlenhydratmischung (TS) aufaddierend, Isomaltose, Glucose, Fructose und/oder Oligomere von Kohlenhydraten aufweist, wobei wenn Oligomere von Kohlenhydraten vorhanden sind, diese in einer Menge von höchstens 0,50 Gew.- % vorliegen (jeweils TS (Trockensubstanz), bezogen auf Gesamt-TS der Kohlenhydratmischung.

Isomalt, insbesondere ein JECFA-gemäßes Isomalt kann in bevorzugter Ausführungsform auch aus einer in Verfahrensschritt a) bereitgestellten Isomaltulose- und Saccharose-haltigen Kohlenhydratmischung hergestellt werden, die 75,00 bis 86,00 Gew.-% Isomaltulose, 13,99 bis 24,99 Gew.-% Trehalulose und höchstens 0,50 Gew.-%, insbesondere 0,01 bis 0,50 Gew.-% Saccharose (jeweils TS bezogen auf Gesamt-TS der Kohlenhydratmischung) aufweist.

Isomalt, insbesondere ein JECFA-gemäßes Isomalt kann in bevorzugter Ausführungsform auch aus einer in Verfahrensschritt a) bereitgestellten Isomaltulose- und Saccharose-haltigen Kohlenhydratmischung hergestellt werden, die 75,00 bis 86,00 Gew.-% Isomaltulose, 13,99 bis 24,99 Gew.-% Trehalulose und höchstens 0,50 Gew.-%, insbesondere 0,01 bis 0,50 Gew.-% Saccharose und, optional auf 100 Gew.-% der Kohlenhydratmischung (TS) aufaddierend, Isomaltose, Glucose, Fructose und/oder Oligomere von Kohlenhydraten aufweist, wobei wenn Oligomere von Kohlenhydraten vorhanden sind, diese in einer Menge von höchstens 0,50 Gew.- % vorliegen (jeweils TS (Trockensubstanz), bezogen auf Gesamt-TS der Kohlenhydratmischung.

Die vorliegende Erfindung betrifft auch die Herstellung eines hochreinen Isomalts, insbesondere das in Verfahrensschritt c) erhaltene Isomalt, welches vorzugsweise mindestens 98,00 Gew.-% hydrierte Mono- und Disaccharide, nämlich 1,6-GPS, 1,1-GPM, 1,1-GPS, Sorbit und Mannit, und höchstens 2,00 Gew.-% Nebenkomponenten aufweist, wobei in dem Isomalt mindestens 98,00 Gew.-% 1,6-GPS und 1,1-GPM, 0 bis 0,50 Gew.-% Sorbit, 0 bis 0,50 Gew.-% Mannit, höchstens 0,50 Gew.-%, insbesondere 0,01 bis 0,50 Gew.-% Saccharose, und 0 bis 0,30 Gew.-%, insbesondere 0,01 bis 0,30 Gew.-% reduzierende Zucker vorhanden sind, wobei einzelne in diesem Isomalt gegebenenfalls vorhandene Nebenkomponenten jeweils in einer Menge von 0 bis 0,50 Gew.-% vorhanden sind, und wobei die Summe aller reduzierenden und nicht-reduzierenden Zucker höchstens 0,50 Gew.-% beträgt (TS, Trockensubstanz jeweils bezogen auf Gesamt-TS Isomalt).

Ein hochreines Isomalt lässt sich in bevorzugter Ausführungsform aus einer in Verfahrensschritt a) bereitgestellten Isomaltulose- und Saccharose-haltigen Kohlenhydratmischung herstellen, die 98,00 bis 99,99 Gew.-% Isomaltulose und höchstens 0,50 Gew.-%, insbesondere 0,01 bis 0,50 Gew.-% Saccharose aufweist (jeweils TS (Trockensubstanz), bezogen auf Gesamt-TS der Kohlenhydratmischung).

Ein derartiges hochreines Isomalt lässt sich in bevorzugter Ausführungsform aus einer in Verfahrensschritt a) bereitgestellten Isomaltulose- und Saccharose-haltigen Kohlenhydratmischung herstellen, die 98,00 bis 99,99 Gew.-% Isomaltulose und höchstens 0,50 Gew.-%, insbesondere 0,01 bis 0,50 Gew.-% Saccharose und optional, auf 100 Gew.-% der Kohlenhydratmischung (TS) aufaddierend, Trehalulose, Isomaltose, Glucose, Fructose und/oder Oligomere von Kohlenhydraten aufweist, wobei wenn Glucose, Fructose und/oder Oligomere von Kohlenhydraten vorhanden sind, diese jeweils in einer Menge von höchstens 0,5 Gew.-% vorliegen (jeweils TS (Trockensubstanz), bezogen auf Gesamt-TS der Kohlenhydratmischung).

In bevorzugter Ausführungsform ist dieses hochreine Isomalt ein 1,6-GPS- und 1,1-GPM-haltiges Gemisch mit einem 1,6-GPS- zu 1,1-GPM-Verhältnis von >1, insbesondere 55 bis 62 Gew.-% 1,6-GPS und 38 bis 45 Gew.-% 1,1-GPM (bezogen auf TS der Gesamtmenge von 1,6-GPS und 1,1-GPM in Isomalt).

In einer besonders bevorzugten Ausführungsform enthält das in Verfahrensschritt c) erhaltene JEFCA-gemäße oder hochreine Isomalt mindestens 98,80 Gew.-%, vorzugsweise mindestens 99,00 Gew.-%, vorzugsweise mindestens 99,10 Gew.-% hydrierte Mono- und Disaccharide, insbesondere Disaccharid-Alkohole (jeweils bezogen auf TS des Isomalts).

In besonders bevorzugter Ausführungsform weist das bereitgestellte Isomalt mindestens 98,00, insbesondere mindestens 98,20 Gew.-%, vorzugsweise mindestens 98,50 Gew.-%, vorzugsweise mindestens 98,60 Gew.-%, vorzugsweise mindestens 98,70 Gew.-% 1,6-GPS, 1,1-GPM und 1,1-GPS, vorzugsweise 1,6-GPS und 1,1-GPM (jeweils bezogen auf Gesamttrockengewicht des Isomalts) auf.

In einer bevorzugten Ausführungsform weist das in Verfahrensschritt c) erhaltene Isomalt vorzugsweise 0,01 bis 0,45 Gew.-%, insbesondere von 0,01 bis 0,42 Gew.-% Saccharose, insbesondere von 0,01 bis 0,49 Gew.-%, insbesondere 0,01 bis 0,20 Gew.-%, insbesondere 0,01 bis 0,04 Gew.-%, insbesondere 0,10 bis 0,50 Gew.-%, insbesondere 0,02 bis 0,04 Gew.-%, insbesondere 0,02 bis 0,03 Gew.-% Saccharose auf (jeweils bezogen auf Gesamttrockengewicht des Isomalts).

In einer besonders bevorzugten Ausführungsform weist das in Verfahrensschritt c) erhaltene Isomalt mindestens 98,00 Gew.-%, vorzugsweise mindestens 98,20 Gew.-%, vorzugsweise mindestens 98,50 Gew.-% Disaccharidalkohole, insbesondere 1,6-GPS und 1,1-GPM sowie optional 1,1-GPS, und 0,01 bis 0,40 Gew.-%, vorzugsweise 0,01 bis 0,03 Gew.-% Saccharose, 0,01 bis 0,30 Gew.-% reduzierende Zucker, höchstens 0,50 Gew.-% Sorbit und höchstens 0,50 Gew.-% Mannit auf, wobei die Summe aller Zucker, insbesondere Isomaltulose, Isomaltose, Saccharose, Fructose und Glucose höchstens 0,50 Gew.-% beträgt (TS, Trockensubstanz jeweils bezogen auf TS Isomalt) beträgt.

In einer Ausführungsform weist das in Verfahrensschritt c) erhaltene Isomalt vorzugsweise mindestens 98,00 Gew.-% 1,6-GPS und 1,1-GPM sowie 0,01 bis 0,05 Gew.-% Saccharose auf (jeweils TS, jeweils bezogen auf TS Isomalt).

Besonders vorteilhaft wird in einer bevorzugten Ausführungsform Isomalt bereitgestellt, das angereichert an 1,6-GPS ist. 1,6-GPS weist im Verhältnis zu 1,1-GPM eine stärkere Süßkraft und ein höhere Löslichkeit in Wasser auf. In einer besonders bevorzugten Ausführungsform der vorliegenden Erfindung beträgt das Gewichtsverhältnis von 1,6-GPS zu 1,1-GPM im erhaltenen Isomalt > 1.

In einer besonders bevorzugten Ausführungsform ist das in Verfahrensschritt c) bereitgestellte Isomalt, insbesondere das bereitgestellte JECFA-gemäße Isomalt, insbesondere das hochreine Isomalt, ein Isomalt mit einem 1,6-GPS- zu 1,1-GPM-Verhältnis von >1, nämlich 55 bis 62 Gew.-% 1,6-GPS und 38 bis 45 Gew.-% 1,1-GPM (bezogen auf TS der Gesamtmenge von 1,6-GPS und 1,1-GPM im Isomalt).

In einer besonders bevorzugten Ausführungsform wird in Verfahrensschritt c) ein 1,6-GPS-angereichertes Isomalt bereitgestellt, insbesondere ein solches, das mehr als 57,00 bis 99,00 Gew.-%, insbesondere 58,00 bis 99,00 Gew.-%, 1,6-GPS und weniger als 43,00 bis 1,00 Gew.- %, insbesondere 42,00 bis 1,00 Gew.-%, 1,1-GPM, insbesondere 75,00 bis 80,00 Gew.-% 1,6-GPS und 25,00 bis 20,00 Gew.-% 1,1-GPM (jeweils bezogen auf Trockensubstanz (TS) der Gesamtmenge an 1,6-GPS und 1,1-GPM) enthält.

In einer weiteren bevorzugten Ausführungsform wird in Verfahrensschritt c) Isomalt bereitgestellt, das 43,00 bis 57,00 Gew.-% 1,6-GPS und 57,00 bis 43,00 Gew.-% 1,1-GPM (jeweils bezogen auf Trockensubstanz (TS) der Gesamtmenge an 1,6-GPS und 1,1-GPM) enthält.

In einer besonders bevorzugten Ausführungsform weist das in Verfahrensschritt c) bereitgestellte Isomalt keine Glucose auf.

In einer besonders bevorzugten Ausführungsform weist das bereitgestellte Isomalt keine Fructose auf.

In einer bevorzugten Ausführungsform weist das bereitgestellte Isomalt kein Sorbit auf. In einer bevorzugten Ausführungsform weist das erfindungsgemäße Isomalt kein Mannit auf.

In einer bevorzugten Ausführungsform weist das bereitgestellte Isomalt kein Sorbit, kein Mannit, keine Glucose und keine Fructose auf.

In einer bevorzugten Ausführungsform der Erfindung weist das in Verfahrensschritt c) bereitgestellte Isomalt weniger als 0,01 Gew.-%, insbesondere keine Isomaltulose auf.

In einer besonders bevorzugten Ausführungsform weist das in Verfahrensschritt c) erhaltene Isomalt höchstens 0,5 Gew.-%, insbesondere höchstens 0,2 Gew.-%, insbesondere höchstens 0,15 Gew.-%, insbesondere höchstens 0,1 Gew.-%, insbesondere höchstens 0,05 Gew.-%, insbesondere höchstens 0,01 Gew.-% Glucose auf (bezogen auf das Gesamtgewicht der Trockensubstanz des Isomalts).

In einer besonders bevorzugten Ausführungsform weist das bereitgestellte Isomalt höchstens 0,5 Gew.-%, insbesondere höchstens 0,2 Gew.-%, insbesondere höchstens 0,15 Gew.-%, insbesondere höchstens 0,1 Gew.-%, insbesondere höchstens 0,05 Gew.-%, insbesondere höchstens 0,01 Gew.-% Fructose auf (bezogen auf das Gesamtgewicht der Trockensubstanz des Isomalts).

In einer bevorzugten Ausführungsform weist das in Verfahrensschritt c) erhaltene Isomalt höchstens 0,5 Gew.-%, insbesondere höchstens 0,2 Gew.-%, insbesondere höchstens 0,15 Gew.- %, insbesondere höchstens 0,1 Gew.-%, insbesondere höchstens 0,05 Gew.-%, insbesondere höchstens 0,01 Gew.-% Sorbit auf (bezogen auf das Gesamtgewicht der Trockensubstanz des Isomalts).

In einer bevorzugten Ausführungsform weist das in Verfahrensschritt c) erhaltene Isomalt höchstens 0,5 Gew.-%, insbesondere höchstens 0,2 Gew.-%, insbesondere höchstens 0,15 Gew.- %, insbesondere höchstens 0,1 Gew.-%, insbesondere höchstens 0,05 Gew.-%, insbesondere höchstens 0,01 Gew.-% Mannit auf (bezogen auf das Gesamtgewicht der Trockensubstanz des Isomalts).

In einer bevorzugten Ausführungsform weist das in Verfahrensschritt c) erhaltene Isomalt höchstens 0,2 Gew.-%, insbesondere höchstens 0,1 Gew.-%, insbesondere höchstens 0,05 Gew.- %, Sorbit, höchstens 0,2 Gew.-%, insbesondere höchstens 0,1 Gew.-%, insbesondere höchstens 0,05 Gew.-%, Mannit, höchstens 0,2 Gew.-%, insbesondere höchstens 0,1 Gew.-%, insbesondere höchstens 0,05 Gew.-%, Glucose und höchstens 0,2 Gew.-%, insbesondere höchstens 0,1 Gew.-%, insbesondere höchstens 0,05 Gew.-%, Fructose auf, wobei die Summe der im Isomalt enthaltenen Zucker, insbesondere Isomaltulose, Isomaltose, Saccharose, Glucose und Fructose, höchstens 0,50 Gew.-% (jeweils bezogen auf das Gesamtgewicht der Trockensubstanz des Isomalts) beträgt.

In einer besonders bevorzugten Ausführungsform der vorliegenden Erfindung weist das Isomalt, erhalten in Verfahrensschritt c), 1,6-GPS, 1,1-GPM und mindestens eine weitere Verbindung auf, ausgewählt aus der Gruppe bestehend aus α-D-Glucopyranosyl-1,1-D-sorbit (1,1-GPS), Sorbit und Mannit. In bevorzugter Ausführungsform stammt dieses in dem Isomalt enthaltene Sorbit und Mannit nicht aus einer Saccharoseumsetzung in Verfahrensschritt b), sondern vielmehr aus optional in der eingesetzten Kohlenhydratmischung gemäß Verfahrensschritt a) enthaltenen Glucose und Fructose.

In einer besonders bevorzugten Ausführungsform der vorliegenden Erfindung enthält das Isomalt, erhalten in Verfahrensschritt c), 50,00 bis 60,00 Gew.-% 1,6-GPS, 35,00 bis 45,00 Gew.-% 1,1-GPM und optional 0,10 bis 15,00, insbesondere 0,10 bis 1,50 Gew.-%, insbesondere 0,10 bis 1,00 Gew.-% 1,1-GPS, 0,00 bis 0,50 Gew.-% Mannit, vorzugsweise kein Mannit, 0,00 bis 0,50 Gew.-% Sorbit, vorzugsweise kein Sorbit und 0,01 bis 0,50 Gew.-% Saccharose, insbesondere 0,01 bis 0,40 Gew.-%, insbesondere 0,01 bis 0,30 Gew.-%, insbesondere 0,01 bis 0,20 Gew.-% , insbesondere 0,01 bis 0,04 Gew.-%, insbesondere 0,02 bis 0,03 Gew.-%, insbesondere 0,03 bis 0,04 Gew.-%, Saccharose, bevorzugt besteht daraus.

In einer besonders bevorzugten Ausführungsform der vorliegenden Erfindung wird die Konzentration an Saccharose in der in Schritt a) bereitgestellten Isomaltulose- und Saccharose-haltigen Kohlenhydratmischung bis zu dem in Schritt c) erhaltenen Isomalt allein durch Einstellung der in Schritt b) definierten Verfahrensparameter konstant gehalten. In einer besonders bevorzugten Ausführungsform der vorliegenden Erfindung wird ein Verfahren zur Herstellung von Isomalt bereitgestellt, im Rahmen dessen die Konzentration an Saccharose in der in Verfahrensschritt a) bereitgestellten Isomaltulose- und Saccharose-haltigen Kohlenhydratmischung im Verfahrensschritt b), insbesondere über das gesamte Verfahren, konstant gehalten wird, insbesondere der Saccharose-Gehalt im erhaltenen Isomalt genauso hoch ist wie der Saccharose-Gehalt in der bereitgestellten Isomaltulose- und Saccharose-haltigen Kohlenhydratmischung.

In einer besonders bevorzugten Ausführungsform der vorliegenden Erfindung wird die Konzentration an Saccharose zwischen der, in Verfahrensschritt a) bereitgestellten, Kohlenhydratmischung und dem, in Verfahrensschritt c) erhaltenen, Isomalt konstant gehalten, das heißt Saccharose wird in Verfahrensschritt b) nicht gespalten und insbesondere nicht gespalten und hydriert.

In einer besonders bevorzugten Ausführungsform der vorliegenden Erfindung wird die Konzentration an Saccharose zwischen der, in Schritt a) bereitgestellten, Kohlenhydratmischung und dem, in Schritt c) erhaltenen, Isomalt allein durch Einstellen der Verfahrensparameter, das heißt Wasserstoffdruck, Reaktionstemperatur, Raumgeschwindigkeit und pH-Wert, konstant gehalten, das heißt Saccharose wird in Verfahrensschritt b) nicht gespalten, insbesondere nicht gespalten und nicht hydriert.

Im Zusammenhang mit der vorliegenden Erfindung wird somit unter "konstant halten" verstanden, dass die Kohlenhydratmischung, bereitgestellt in Verfahrensschritt a), vor der Umsetzung in Verfahrensschritt b) den gleichen Gehalt an Saccharose aufweist, wie das nach der Umsetzung in Verfahrensschritt c) erhaltene Isomalt. In Verfahrensschritt b) wird daher die Saccharose nicht gespalten, insbesondere nicht gespalten und nicht hydriert. In besonders bevorzugter Ausführungsform wird der Saccharosegehalt als konstant angesehen, wenn sich unter Verwendung einer GC-Analysen-Methode (GC-FID) mit einer Bestimmungsgrenze von 0,01g Saccharose/100g Trockensubstanz keine Änderung des Saccharosegehaltes während der Umsetzung gemäß Verfahrensschritt b) ergibt. Erfindungsgemäß besonders bevorzugt wird zur Bestimmung der Saccharosegehalte vor und nach Umsetzung gemäß Verfahrensschritt b) der Saccharosegehalt (die beiden erhaltenen Werte bilden ein Messpaar) jeweils mehrfach, insbesondere 4-mal, bestimmt und ein Mittelwert für den Saccharosegehalt vor und ein Mittelwert für den Saccharosegehalt nach Durchführung des Verfahrensschritts b) bestimmt (Mittelwertbestimmung mittels upper bound-Verfahren).

Der Saccharosegehalt gilt in bevorzugter Ausführungsform dann als konstant gehalten, wenn der Mittelwert des Gehalts an Saccharose in der Kohlenhydratmischung dem Gehalt an Saccharose im erhaltenen Isomalt entspricht oder wenn mittels Differenzen-t-Test keine signifikante Differenz zwischen den Mittelwerten der Gehalte an Saccharose in der Kohlenhydratmischung und im erhaltenen Isomalt festgestellt wird.

Für die Berechnung mittels Differenzen-t-Test wird unter Nutzung der erhaltenen Mittelwerte und Messwerte folgende Formel verwendet:

$$\tau = \left| \frac{\bar{x}_A - \bar{x}_B}{\sqrt{\frac{1}{n-1} \cdot \sum (\Delta x_i - \Delta \bar{x})^2}} \right| \cdot \sqrt{n}$$

wobei ($\bar{x}_A$) der Mittelwert des Komponentengehaltes in der Kohlenhydratmischung, ($\bar{x}_B$) der Mittelwert des Komponentengehaltes im Isomalt, ($\Delta\bar{x}$) die Differenz der Mittelwerte ($\bar{x}_A$ - $\bar{x}_B$), ($\Delta x_i$) die Differenz der jeweiligen Messpaare A (Kohlenhydratmischung) - B (Isomalt), (n) die Anzahl der Messpaare der Kohlenhydratmischung und des Isomalt und ($\tau$) die Prüfgröße ist.

Eine signifikante Differenz (99,9 % Signifikanzniveau) zwischen den Mittelwerten der Gehalte an Saccharose in der Kohlenhydratmischung und im Isomalt liegt bei n = 4 dann vor, wenn die Prüfgröße ($\tau$) größer als (t) = 12,924 (99,9 % Signifikanzniveau, 3 Freiheitsgrade) ist.

Ein optionales Abtrennen von Saccharose, insbesondere in einen Verfahrensschritt a0), vor der Durchführung von Verfahrensschritt b) bleibt davon unberührt.

In einer besonders bevorzugten Ausführungsform der vorliegenden Erfindung kann das Isomalt verwendet werden zur Herstellung von Produkten für den menschlichen und/oder tierischen Verzehr oder von pharmazeutischen Produkten.

Die vorliegende Erfindung betrifft daher auch Produkte für den menschlichen und/oder tierischen Verzehr oder pharmazeutische Produkte enthaltend erfindungsgemäßes Isomalt.

In einer bevorzugten Ausführungsform ist das Produkt für den menschlichen Verzehr ein Nahrungs- oder Genussmittel wie beispielsweise eine Süßware, eine Füllung für Süßwaren, Hart- und/oder Weichkaramellen, ein Fondant, ein Joghurt, ein Gebäck, ein Kaugummi, eine Eiscreme, ein Milchprodukt, eine Fruchtzubereitung, eine Marmelade, ein Gelee oder ein Smoothie.

Hartkaramellen aus mindestens 96,00 Gew.-% erfindungsgemäßem Isomalt (bezogen auf das Gesamtgewicht der Hartkaramelle) weisen bei drei Tagen offener Lagerung bei 30 °C und 65 % relativer Luftfeuchte eine Wasseraufnahme von maximal 1,30 Gew.-%, insbesondere 1,20 Gew.-%, insbesondere 1,10 Gew.-%, insbesondere 1,00 Gew.-%, insbesondere 0,90 Gew.-%, insbesondere 0,80 Gew.-% (jeweils bezogen auf Gesamtgewicht der Hartkaramelle) auf.

Hartkaramellen aus mindestens 96,00 Gew.-% erfindungsgemäßem Isomalt (bezogen auf das Gesamtgewicht der Hartkaramelle) weisen bei drei Tagen offener Lagerung bei 25 °C und 80 % relativer Luftfeuchte eine maximale Wasseraufnahme von maximal 6,0 Gew.-%, insbesondere 5,50 Gew.-%, insbesondere 5,00 Gew.-%, insbesondere 4,50 Gew.-%, insbesondere 4,00 Gew.-%, insbesondere 3,50 Gew.-%, insbesondere 3,00 Gew.-% (jeweils bezogen auf Gesamtgewicht der Hartkaramelle) auf.

Die vorliegende Erfindung betrifft daher auch Hartkaramellen enthaltend erfindungsgemäßes Isomalt, insbesondere enthaltend mindestens 96,00 Gew.-% erfindungsgemäßes Isomalt (bezogen auf das Gesamtgewicht der Hartkaramellen), insbesondere Hartkaramellen, die sich durch die vorstehend angegebenen maximalen Wasseraufnahmen bei den angegebenen Bedingungen auszeichnen.

Weitere vorteilhafte Ausgestaltungen ergeben sich aus den Unteransprüchen.

Die Erfindung wird anhand des nachfolgenden Beispiels näher erläutert.

Beispiel

Hydrierung von Isomaltulose-, Trehalulose- und Saccharose-haltigen Lösungen

Die Hydrierungen erfolgten in einem kontinuierlichen Hochdruckreaktor im Trickle bed-Verfahren. Die jeweiligen in Tabelle 1, nachstehend, identifizierten Kohlenhydratmischungen wurden in Wasser gelöst und vortemperiert. Als Katalysator wurden 1,5 % Ru/Al$_2$O$_3$-Kugeln eingesetzt. Die im Reaktor verwendeten Reaktionsbedingungen Druck, pH-Wert, LHSV und Reaktionstemperatur sowie die Gehalte an Trockensubstanz von Isomaltulose, Trehalulose und Saccharose sowie Glucose und Fructose an der Gesamt-TS (Trockensubstanz) der zu hydrierenden Kohlenhydratmischung (bestimmt mittels GC-FID) sind jeweils in der Tabelle 1 angegeben.

Die gemäß der Reaktionsnummern 1, 2, 3, 4, 5, 6, 7, 8, 9, 10, 11, 12, 13, 14, 15, 16, 17, 18, 19, 20, 21, 22, und 23 eingesetzten Kohlenhydratmischungen wurden vor Eintritt in den Reaktor jeweils auf Temperaturen von 47,4 °C, 48,2 °C, 46,2 °C, 55,5 °C, 58,5 °C, 67,3 °C, 45,0 °C, 54,4 °C, 58,0 °C, 63,5 °C, 66,5 °C, 68,5 °C, 70,9 °C, 103 °C, 103 °C, 103 °C, 103 °C, 68 °C, 103 °C, 67,3 °C, 105 °C, 67 °C und 105 °C vortemperiert und dann bei der in der Tabelle 1 angegebenen Reaktionstemperatur im Reaktor zur Umsetzung gebracht. Reaktionsnummern 14 bis 23 stellen nicht-erfindungsgemäße Vergleichsläufe dar.

Tabelle 1

| Reaktions-Nr. | Wasserstoffdruck [MPa] | pH-Wert [-] | LHSV [h⁻¹] | Reaktionstemperatur [°C] | TS-Isomaltulose [Gew.- %] | TS-Trehalulose [Gew.- %] | TS-Saccharose [Gew.- %] | TS-Fructose [Gew. -%] | TS-Glucose [Gew. -%] | TS ref. [g/100 g] |
|---|---|---|---|---|---|---|---|---|---|---|
| 1 | 16,5 | 3,4 | 0,58 | 71,1 | 99,08 | 0,66 | 0,02 | 0,08 | 0,05 | 41,62 |
| 2 | 16,5 | 4,3 | 0,58 | 70,5 | 98,96 | 0,67 | 0,02 | 0,07 | 0,05 | 40,83 |
| 3 | 16,5 | 5,1 | 0,48 | 69,5 | 98,76 | 0,69 | 0,02 | 0,09 | 0,10 | 40,58 |
| 4 | 16,5 | 4,8 | 0,67 | 77,8 | 98,88 | 0,75 | 0,02 | 0,08 | 0,06 | 41,36 |
| 5 | 16,5 | 4,9 | 0,77 | 81,2 | 98,88 | 0,77 | 0,02 | 0,07 | 0,05 | 41,47 |
| 6 | 16,5 | 5,3 | 0,87 | 91,0 | 98,78 | 0,82 | 0,02 | 0,07 | 0,06 | 41,90 |
| 7 | 17,5 | 3,0 | 0,28 | 69,7 | 99,11 | 0,60 | 0,03 | 0,06 | 0,06 | 41,27 |
| 8 | 17,5 | 4,4 | 0,65 | 80,8 | 99,08 | 0,68 | 0,02 | 0,05 | 0,05 | 41,45 |
| 9 | 17,5 | 5,6 | 0,37 | 83,5 | 98,60 | 0,81 | 0,05 | 0,10 | 0,09 | 40,61 |
| 10 | 17,5 | 5,5 | 0,65 | 90,0 | 98,79 | 0,62 | 0,02 | 0,04 | 0,07 | 41,65 |
| 11 | 17,5 | 5,1 | 0,56 | 92,3 | 98,88 | 0,67 | 0,02 | 0,08 | 0,10 | 41,14 |
| 12 | 17,5 | 5,9 | 0,46 | 95,0 | 98,88 | 0,77 | 0,02 | 0,07 | 0,05 | 41,47 |
| 13 | 17,5 | 5,4 | 0,56 | 98,3 | 98,76 | 0,76 | 0,03 | 0,09 | 0,11 | 41,64 |
| 14 | 17,5 | 5,8 | 0,35 | 110,0 | 98,60 | 0,65 | 0,50 | 0,06 | 0,05 | 41,56 |
| 15 | 17,5 | 5,6 | 0,35 | 110,0 | 99,02 | 0,55 | 0,06 | 0,09 | 0,03 | 41,37 |
| 16 | 17,5 | 5,5 | 0,38 | 120,0 | 98,49 | 0,65 | 0,50 | 0,05 | 0,06 | 41,59 |
| 17 | 17,5 | 3,0 | 0,47 | 120,0 | 91,00 | 0,48 | 6,96 | 0,09 | 0,13 | 42,72 |
| 18 | 5,0 | 3,8 | 0,47 | 91,0 | 92,12 | 0,48 | 7,04 | 0,09 | 0,12 | 42,79 |
| 19 | 5,0 | 4,8 | 0,47 | 120,0 | 99,07 | 0,49 | 0,05 | 0,09 | 0,13 | 42,74 |
| 20 | 5,0 | 4,0 | 0,47 | 89,9 | 98,96 | 0,61 | 0,04 | 0,06 | 0,04 | 42,84 |
| 21 | 6,0 | 2,4 | 0,47 | 121 | 98,96 | 0,59 | 0,03 | 0,07 | 0,05 | 41,36 |
| 22 | 6,0 | 2,6 | 0,47 | 90 | 92,27 | 0,54 | 6,85 | 0,10 | 0,07 | 43,08 |

(fortgesetzt)

| Reaktions-Nr. | Wasserstoffdruck [MPa] | pH-Wert [-] | LHSV $[h^{-1}]$ | Reaktionstemperatur [°C] | TS-Isomaltulose [Gew.- %] | TS-Trehalulose [Gew.- %] | TS-Saccharose [Gew.- %] | TS-Fructose [Gew. -%] | TS-Glucose [Gew. -%] | TS ref. [g/100 g] |
|---|---|---|---|---|---|---|---|---|---|---|
| 23 | 6,0 | 2,5 | 0,47 | 120 | 92,27 | 0,54 | 6,85 | 0,10 | 0,07 | 43,08 |
| (Gew.-%- Angaben jeweils bezogen auf Gesamttrockensubstanz) (TS ref. ist der mittels Refraktometer bestimmte Trockensubstanzgehalt) (Reaktionsnummern 14 bis 23 stellen nicht-erfindungsgemäße Vergleichsläufe dar) | | | | | | | | | | |

Tabelle 2a

| Reaktions-Nr. | TS-1,6-GPS [Gew.-%] | TS-1,1-GPM [Gew.-%] | TS-1,1-GPS [Gew.-%] | TS-Mannit [Gew.-%] | TS-Sorbit [Gew.-%] | TS-Isomaltulose [Gew.-%] | TS-Trehalulose [Gew.-%] | TS-Saccharose [Gew.-%] |
|---|---|---|---|---|---|---|---|---|
| 1 | 58,96 | 39,91 | 0,33 | 0,03 | 0,09 | <0,01 | <0,01 | 0,02 |
| 2 | 58,72 | 39,98 | 0,27 | 0,02 | 0,07 | <0,01 | <0,01 | 0,02 |
| 3 | 59,45 | 39,40 | 0,31 | 0,03 | 0,09 | <0,01 | <0,01 | 0,02 |
| 4 | 58,65 | 40,06 | 0,34 | 0,03 | 0,08 | <0,01 | <0,01 | 0,02 |
| 5 | 58,63 | 40,25 | 0,26 | 0,02 | 0,05 | <0,01 | <0,01 | 0,02 |
| 6 | 57,98 | 40,67 | 0,37 | 0,03 | 0,08 | <0,01 | <0,01 | 0,02 |
| 7 | 58,01 | 41,13 | 0,30 | 0,03 | 0,02 | <0,01 | <0,01 | 0,03 |
| 8 | 57,60 | 41,47 | 0,34 | 0,02 | 0,07 | 0,03 | <0,01 | 0,02 |
| 9 | 57,52 | 41,46 | 0,30 | 0,04 | 0,10 | 0,03 | <0,01 | 0,05 |
| 10 | 56,88 | 42,00 | 0,35 | 0,08 | 0,04 | 0,04 | <0,01 | 0,02 |
| 11 | 56,47 | 42,23 | 0,38 | 0,03 | 0,09 | <0,01 | <0,01 | 0,02 |
| 12 | 56,71 | 41,75 | 0,34 | 0,02 | 0,05 | <0,01 | <0,01 | 0,02 |
| 13 | 56,11 | 42,15 | 0,41 | 0,04 | 0,10 | <0,01 | <0,01 | 0,03 |
| 14 | 49,24 | 43,55 | 1,66 | 0,20 | 0,28 | <0,01 | <0,01 | 0,22 |
| 15 | 49,11 | 43,95 | 1,73 | 0,05 | 0,12 | <0,01 | <0,01 | 0,02 |
| 16 | 48,16 | 43,10 | 1,90 | 0,19 | 0,30 | <0,01 | <0,01 | 0,18 |
| 17 | 42,29 | 40,22 | 1,78 | 1,87 | 4,27 | <0,01 | <0,01 | 0,24 |
| 18 | 52,20 | 39,78 | 0,18 | 1,07 | 3,31 | 0,05 | <0,01 | 2,63 |
| 19 | 48,05 | 39,73 | 1,78 | 0,04 | 0,16 | <0,01 | <0,01 | 0,02 |
| 20 | 55,1 | 41,78 | 0,36 | 0,03 | 0,10 | 0,01 | <0,01 | 0,01 |
| 21 | 43,82 | 40,20 | 2,36 | 0,49 | 1,01 | <0,01 | <0,01 | <0,01 |
| 22 | 51,63 | 39,44 | 0,43 | 0,41 | 1,48 | <0,01 | <0,01 | 4,66 |
| 23 | 42,49 | 39,40 | 1,95 | 1,79 | 4,30 | <0,01 | <0,01 | 0,02 |

(Gew.-%- Angaben jeweils bezogen auf Gesamttrockensubstanz)
(Reaktionsnummern 14 bis 23 stellen nicht-erfindungsgemäße Vergleichsläufe dar)

Tabelle 2b

| Reaktions-Nr. | TS-Glucose [Gew.-%] | TS-Fructose [Gew.-%] | TS ref. [g/100 g] | Summe TS 1,1-GPM und 1,6-GPS [Gew.-%] | Summe TS hydrierte Mono- und Disaccharide [Gew.-%] | Summe Nebenkomponenten [Gew.-%] | Isomaltulose-Umsatz [mol-%] | Selektivität Isomalt [1,1-GPM und 1,6-GPS) [mol-%] |
|---|---|---|---|---|---|---|---|---|
| 1 | <0,01 | <0,01 | 41,90 | 98,87 | 99,32 | 0,68 | >99,99 | 99,20 |
| 2 | <0,01 | <0,01 | 40,98 | 98,70 | 99,06 | 0,94 | >99,99 | 99,15 |
| 3 | <0,01 | <0,01 | 41,13 | 98,85 | 99,29 | 0,71 | >99,99 | 99,51 |
| 4 | <0,01 | <0,01 | 41,66 | 98,71 | 99,15 | 0,85 | >99,99 | 99,24 |
| 5 | <0,01 | <0,01 | 41,21 | 98,88 | 99,21 | 0,79 | >99,99 | 99,41 |
| 6 | <0,01 | <0,01 | 41,79 | 98,65 | 99,12 | 0,88 | >99,99 | 99,28 |
| 7 | <0,01 | <0,01 | 41,70 | 99,14 | 99,55 | 0,45 | >99,99 | 99,44 |
| 8 | <0,01 | <0,01 | 41,34 | 99,07 | 99,50 | 0,50 | 99,97 | 99,40 |
| 9 | <0,01 | <0,01 | 40,96 | 98,98 | 99,42 | 0,58 | >99,99 | 99,80 |
| 10 | <0,01 | <0,01 | 41,77 | 98,88 | 99,34 | 0,66 | 99,96 | 99,51 |
| 11 | <0,01 | <0,01 | 41,68 | 98,70 | 99,20 | 0,80 | >99,99 | 99,23 |
| 12 | <0,01 | <0,01 | 41,06 | 98,46 | 98,88 | 1,12 | >99,99 | 98,99 |
| 13 | <0,01 | <0,01 | 41,96 | 98,26 | 98,81 | 1,19 | >99,99 | 98,91 |
| 14 | <0,01 | 0,01 | 42,34 | 92,79 | 94,93 | 5,07 | >99,99 | 93,56 |
| 15 | <0,01 | <0,01 | 42,27 | 93,06 | 94,96 | 5,04 | >99,99 | 93,43 |
| 16 | <0,01 | <0,01 | 42,52 | 91,26 | 93,65 | 6,35 | >99,99 | 92,12 |
| 17 | 0,11 | 0,03 | 43,04 | 82,51 | 89,75 | 10,25 | >99,99 | 90,14 |
| 18 | 0,04 | 0,20 | 44,49 | 91,98 | 96,54 | 3,46 | 99,95 | 99,26 |
| 19 | <0,01 | <0,01 | 43,04 | 87,78 | 89,76 | 10,24 | >99,99 | 88,09 |
| 20 | <0,01 | <0,01 | 43,18 | 96,88 | 97,35 | 2,65 | 99,99 | 97,32 |
| 21 | 0,10 | 0,03 | 41,96 | 84,01 | 87,87 | 12,13 | >99,99 | 84,40 |
| 22 | 0,19 | 0,23 | 43,73 | 91,07 | 93,39 | 6,61 | >99,99 | 98,12 |

| Reaktions-Nr. | TS-Glucose [Gew.- %] | TS-Fructose [Gew.- %] | TS ref. [g/100 g] | Summe TS 1,1-GPM und 1,6-GPS [Gew.-%] | Summe TS hydrierte Mono- und Disaccharide [Gew.-%] | Summe Nebenkomponenten [Gew.-%] | Isomaltulose-Umsatz [mol-%] | Selektivität Isomalt [1,1-GPM und 1,6-GPS) [mol-%] |
|---|---|---|---|---|---|---|---|---|
| 23 | 0,04 | 0,04 | 43,64 | 81,89 | 89,94 | 10,06 | <99,99 | 88,23 |

(Gew.-%- Angaben jeweils bezogen auf Gesamttrockensubstanz,

Reaktionsnummern 14 bis 23 stellen nicht-erfindungsgemäße Vergleichsläufe dar.

Für alle Reaktionsläufe wurde die Berechnung der Summe hydrierter Mono- und Disaccharide gemäß der Joint FAO/WHO Expert Committee on Food Additives (JECFA), Spezifikation für Isomalt (69. JECFA (2008), publiziert in FAO JECFA Monographs 5 (2008)) berechnet. Die Summe erfasst die aufaddierten Gewichtsanteile von 1,1-GPM, 1,6-GPS, 1,1-GPS, Mannit und Sorbit.

Die Summe der Nebenkomponenten ist die Differenz von 100 - Summe der hydrierten Mono- und Disaccharide.

TS ref. ist der mittels Refraktometer bestimmte Trockensubstanzgehalt.)

Tabellen 2a und 2b lassen sich die Zusammensetzungen der erhaltenen Isomalte entnehmen.

Es zeigt sich, dass im Vergleich zu den nicht-erfindungsgemäß hergestellten Produkten der Reaktionen 14 bis 23 in den Reaktionen 1 bis 13 ein besonders reines Isomalt hergestellt wurde. Es zeigt sich darüber hinaus, dass überraschenderweise der Saccharosegehalt ausgehend vom Saccharosegehalt der in den Reaktionsnummern 1 bis 13 eingesetzten Kohlenhydratmischungen bis zum erhaltenen Isomalt konstant blieb, wohingegen der Gehalt an 1,6-GPS, 1,1-GPM und 1,1-GPS zu- und der Gehalt an Isomaltulose abnahm.

**Patentansprüche**

1. Verfahren zur kontinuierlichen Herstellung von Isomalt aus einer Isomaltulose- und Saccharose-haltigen Kohlenhydratmischung, umfassend die Verfahrensschritte

   a) Bereitstellung einer in wässrigem Medium vorliegenden Isomaltulose- und Saccharose-haltigen Kohlenhydratmischung, enthaltend 75,00 bis 99,99 Gew.-% Isomaltulose und 0,01 bis 0,50 Gew.-% Saccharose (jeweils TS (Trockensubstanz), bezogen auf Gesamt-TS der Kohlenhydratmischung), von Wasserstoff und eines Ruthenium-basierten Katalysators,
   b) Umsetzung der Kohlenhydratmischung zu Isomalt durch kontinuierliches Inkontaktbringen der im wässrigen Medium vorliegenden Kohlenhydratmischung mit dem Ruthenium-basierten Katalysator und Wasserstoff mit einer Raumgeschwindigkeit von 0,25 bis 1,5 h$^{-1}$, bei einem Wasserstoffdruck von 16,0 bis 22,0 MPa und einem pH-Wert von 2,0 bis 6,0 zum Erhalt eines Isomalt-haltigen Produktstroms unter Einstellen einer Reaktionstemperatur von höchstens 100 °C und
   c) Erhalt des Isomalts.

2. Verfahren nach Anspruch 1, wobei die in Verfahrensschritt a) bereitgestellte Isomaltulose- und Saccharose-haltige Kohlenhydratmischung durch enzymatische Umsetzung von Saccharose oder einer Saccharose-haltigen Ausgangsmischung mit einer Saccharose-Glucosylmutase erhalten wurde.

3. Verfahren nach Anspruch 2, wobei die durch enzymatische Umsetzung mit einer Saccharose-Glucosylmutase aus Saccharose oder einer Saccharose-haltigen Ausgangsmischung erhaltene Isomaltulose- und Saccharose-haltige Kohlenhydratmischung einem Verfahrensschritt a0) zur Reduzierung des Saccharosegehaltes auf einen Gehalt von 0,01 bis 0,50 Gew.-% Saccharose (Trockensubstanz bezogen auf Gesamttrockensubstanz der Kohlenhydratmischung) unterzogen wird.

4. Verfahren nach einem der Ansprüche 1 bis 3, wobei in Verfahrensschritt b) eine Reaktionstemperatur von 70 °C bis 95 °C, insbesondere 70 °C bis 91 °C eingestellt wird.

5. Verfahren nach einem der vorhergehenden Ansprüche, wobei nach Verfahrensschritt a) und vor Verfahrensschritt b) die in Verfahrensschritt a) bereitgestellte Kohlenhydratmischung in einem Verfahrensschritt a1) vortemperiert wird, insbesondere auf eine Temperatur von 30 bis 80 °C, insbesondere 30 bis 75 °C, insbesondere 30 bis 70 °C.

6. Verfahren nach einem der vorhergehenden Ansprüche, wobei die Isomaltulose in Verfahrensschritt b) mit einer Umsatzrate von 99 bis 100 mol-%, insbesondere 99,5 bis 100 mol-% umgesetzt wird.

7. Verfahren nach einem der vorhergehenden Ansprüche, wobei die Isomaltulose in Verfahrensschritt b) mit einer Selektivität von 97 bis 100 mol-%, insbesondere 98 bis 100 mol-% zu 1,6-GPS (6-O-α-D-Glucopyranosyl-D-sorbit) und 1,1-GPM (1-O-α-D-Glucopyranosyl-D-mannit) umgesetzt wird.

8. Verfahren nach einem der vorhergehenden Ansprüche, wobei die in Verfahrensschritt a) bereitgestellte Isomaltulose- und Saccharose-haltige Kohlenhydratmischung 86 bis 99,99 Gew.-% Isomaltulose, insbesondere 98 bis 99,99 Gew.-% Isomaltulose, und 0,01 bis 0,50 Gew.-% Saccharose (jeweils TS bezogen auf Gesamt-TS der Kohlenhydratmischung) aufweist.

9. Verfahren nach einem der Ansprüche 1 bis 7, wobei die in Verfahrensschritt a) bereitgestellte Isomaltulose- und Saccharose-haltige Kohlenhydratmischung 75,00 bis 99,49 Gew.-% Isomaltulose, 0,30 bis 24,99 Gew.-% Trehalulose und 0,01 bis 0,50 Gew.-% Saccharose (jeweils TS bezogen auf Gesamt-TS der Kohlenhydratmischung) aufweist.

10. Verfahren nach einem der vorhergehenden Ansprüche 1 bis 7 und 9, wobei die in Schritt a) bereitgestellte Kohlenhydratmischung 75 bis 86,00 Gew.-% Isomaltulose, 0,01 bis 0,50 Gew.-% Saccharose und 13,99 bis 24,99 Gew.-% Trehalulose (jeweils bezogen auf Trockensubstanz der Kohlenhydratmischung), aufweist.

11. Verfahren nach einem der vorhergehenden Ansprüche 1 bis 10, wobei der Trockensubstanzgehalt der in wässrigem Medium vorliegenden Kohlenhydratmischung 35 bis 45 Gew.- % (bezogen auf Gesamtgewicht des Mediums) beträgt.

12. Verfahren nach einem der vorhergehenden Ansprüche, wobei der Ruthenium-basierte Katalysator ein auf einem Träger immobilisierter Katalysator ist.

13. Verfahren nach einem der vorhergehenden Ansprüche, wobei der Ruthenium-basierte Katalysator ein monometallischer oder ein bimetallischer Katalysator ist.

14. Verfahren nach Anspruch 12 oder 13, wobei der Träger Kohlenstoff, ein Metalloxid, insbesondere $Al_2O_3$, $TiO_2$, $SiO_2$, $ZrO_2$ oder ein Zeolith ist.

15. Verfahren nach einem der vorhergehenden Ansprüche, wobei der Rutheniumgehalt des Katalysators 0,05 bis 5,00 Gew.-% beträgt.

16. Verfahren nach einem der vorhergehenden Ansprüche, wobei der pH-Wert in Verfahrensschritt b) 2,0 bis 5,5 beträgt.

17. Verfahren nach einem der vorhergehenden Ansprüche, wobei die Konzentration an Saccharose in der in Schritt a) bereitgestellten Kohlenhydratmischung bis zu dem in Schritt c) erhaltenen Isomalt allein durch Einstellung der im Schritt b) definierten Verfahrensparameter konstant gehalten wird.

18. Verfahren nach einem der vorhergehenden Ansprüche, wobei das in Schritt c) erhaltene Isomalt mindestens 98,00 Gew.-% hydrierte Mono- und Disaccharide, mindestens 86,00 Gew.-% 1,6-GPS und 1,1-GPM, 0,01 bis 0,50 Gew.-% Saccharose und höchstens 0,30 Gew.-% reduzierende Zucker aufweist (jeweils TS (Trockensubstanz), jeweils bezogen auf TS Isomalt).

19. Verfahren nach einem der vorhergehenden Ansprüche, wobei das in Schritt c) erhaltene Isomalt mindestens 98,00 Gew.-% hydrierte Mono- und Disaccharide, mindestens 98,00 Gew.-% 1,6-GPS und 1,1-GPM, 0,01 bis 0,50 Gew.-% Saccharose, höchstens 0,30 Gew.-% reduzierende Zucker, 0 bis 0,50 Gew.-% Sorbit und 0 bis 0,50 Gew.-% Mannit aufweist, wobei einzelne in diesem Isomalt gegebenenfalls vorhandene Nebenkomponenten jeweils in einer Menge von 0 bis 0,50 Gew.-% vorhanden sind und wobei die Summe aller reduzierenden und nicht-reduzierenden Zucker höchstens 0,50 Gew.-% beträgt (jeweils TS (Trockensubstanz), jeweils bezogen auf TS Isomalt).

20. Verfahren nach einem der vorhergehenden Ansprüche, wobei das in Schritt c) erhaltene Isomalt Sorbit-frei ist.

## Claims

1. A method for continuous production of isomalt from an isomaltulose- and sucrose-containing carbohydrate mixture, comprising the method steps

   a) Provision of an isomaltulose- and sucrose-containing carbohydrate mixture present in an aqueous medium, containing 75.00 to 99.99 wt.% isomaltulose and 0.01 to 0.50 wt.% sucrose (each DM (dry matter), based on total DM of the carbohydrate mixture), of hydrogen and a ruthenium-based catalyst,
   b) Conversion of the carbohydrate mixture to isomalt by continuously bringing the carbohydrate mixture present in the aqueous medium into contact with the ruthenium-based catalyst and hydrogen at a space velocity of 0.25 to 1.5 h$^{-1}$, at a hydrogen pressure of 16.0 to 22.0 MPa and a pH of 2.0 to 6.0 to obtain an isomalt-containing product stream while setting a reaction temperature of at most 100 °C and
   c) Obtaining the isomalt.

2. The method of claim 1, wherein the isomaltulose and sucrose-containing carbohydrate mixture provided in method step a) was obtained by enzymatic conversion of sucrose or a sucrose-containing starting mixture with a sucrose-glucosylmutase.

3. The method of claim 2, wherein the isomaltulose and sucrose-containing carbohydrate mixture obtained by enzymatic conversion with a sucrose-glucosyl mutase from sucrose or a sucrose-containing starting mixture is subjected to a method step a0) for reduction of the sucrose content to a content of 0.01 to 0.50 wt.% sucrose (dry matter based on total dry matter of the carbohydrate mixture).

4. The method of one of claims 1 to 3, wherein in method step b) a reaction temperature of 70 °C to 95 °C, in particular 70 °C to 91 °C, is set.

5. The method of one of the preceding claims, wherein after method step a) and before method step b) the carbohydrate mixture provided in method step a) is preheated in a method step a1), in particular to a temperature of 30 to 80 °C, in particular 30 to 75 °C, in particular 30 to 70 °C.

6. The method of one of the preceding claims, wherein the isomaltulose is converted in method step b) at a conversion rate of 99 to 100 mol%, in particular 99.5 to 100 mol%.

7. The method of one of the preceding claims, wherein the isomaltulose in method step b) is converted with a selectivity of 97 to 100 mol%, in particular 98 to 100 mol%, to 1,6-GPS (6-O-$\alpha$-D-glucopyranosyl-D-sorbitol) and 1,1-GPM (1-O-$\alpha$-D-glucopyranosyl-D-mannitol).

8. The method of one of the preceding claims, wherein the isomaltulose- and sucrose-containing carbohydrate mixture provided in method step a) has 86 to 99.99 wt.% isomaltulose, in particular 98 to 99.99 wt.% isomaltulose, and 0.01 to 0.50 wt.% sucrose (each DM based on total DM of the carbohydrate mixture).

9. The method of one of claims 1 to 7, wherein the isomaltulose- and sucrose-containing carbohydrate mixture provided in method step a) has 75.00 to 99.49 wt.% isomaltulose, 0.30 to 24.99 wt.% trehalulose and 0.01 to 0.50 wt.% sucrose (each DM based on total DM of the carbohydrate mixture).

10. Method of one of the preceding claims 1 to 7 and 9, wherein the carbohydrate mixture provided in step a) has 75 to 86.00 wt.% isomaltulose, 0.01 to 0.50 wt.% sucrose and 13.99 to 24.99 wt.% trehalulose (each based on dry matter of the carbohydrate mixture).

11. The method of one of the preceding claims 1 to 10, wherein the dry matter content of the carbohydrate mixture present in the aqueous medium is 35 to 45 wt.% (based on total weight of the medium).

12. The method of one of the preceding claims, wherein the ruthenium-based catalyst is a catalyst immobilized on a carrier.

13. The method of one of the preceding claims, wherein the ruthenium-based catalyst is a monometallic or a bimetallic catalyst.

14. The method of claim 12 or 13, wherein the carrier is carbon, a metal oxide, in particular $Al_2O_3$, $TiO_2$, $SiO_2$, $ZrO_2$ or a zeolite.

15. The method of one of the preceding claims, wherein the ruthenium content of the catalyst is 0.05 to 5.00 wt.%.

16. The method of one of the preceding claims, wherein the pH value in method step b) is 2.0 to 5.5.

17. The method of one of the preceding claims, wherein the concentration of sucrose in the carbohydrate mixture provided in step a) is kept constant up to the isomalt obtained in step c) solely by setting the method parameters defined in step b).

18. The method of one of the preceding claims, wherein the isomalt obtained in step c) has at least 98.00 wt.% hydrogenated mono- and disaccharides, at least 86.00 wt.% 1,6-GPS and 1,1-GPM, 0.01 to 0.50 wt.% sucrose and at most 0.30 wt.% reducing sugars (each DM (dry matter), each based on DM isomalt).

19. The method of one of the preceding claims, wherein the isomalt obtained in step c) has at least 98.00 wt.% hydrogenated mono- and disaccharides, at least 98.00 wt.% 1,6-GPS and 1,1-GPM, 0.01 to 0.50 wt.% sucrose, at most 0.30 wt.% reducing sugars, 0 to 0.50 wt.% sorbitol and 0 to 0.50 wt.% mannitol, wherein single secondary components possibly present in this isomalt are each present in an amount of 0 to 0.50 wt.% and wherein the sum of all reducing and non-reducing sugars is at most 0.50 wt.% (each DM (dry matter), each based on DM isomalt).

20. The method of one of the preceding claims, wherein the isomalt obtained in step c) is sorbitol-free.

**Revendications**

1. Un procédé de production en continu d'isomalt à partir d'un mélange de glucides contenant de l'isomaltulose et du saccharose, comprenant les étapes de procédé

   a) Fourniture d'un mélange de glucides contenant de l'isomaltulose et du saccharose, présent dans un milieu aqueux, contenant 75,00 à 99,99 % en poids d'isomaltulose et 0,01 à 0,50 % en poids de saccharose (chaque MS (matière sèche), par rapport à la MS totale du mélange de glucides), d'hydrogène et d'un catalyseur à base de ruthénium,
   b) Conversion du mélange de glucides en isomalt par mise en contact continue du mélange de glucides présent dans le milieu aqueux avec le catalyseur à base de ruthénium et de l'hydrogène à une vitesse spatiale de 0,25 à 1,5 $h^{-1}$, à une pression d'hydrogène de 16,0 à 22,0 MPa et à un pH de 2,0 à 6,0 pour obtenir un courant de produit contenant de l'isomalt, tout en réglant la température de réaction à au moins 100 °C, et
   c) Obtenir l'isomalt.

2. Le procédé selon la revendication 1, dans lequel le mélange de glucides contenant de l'isomaltulose et du saccharose fourni dans l'étape de procédé a) a été obtenu par conversion enzymatique du saccharose ou d'un mélange initial contenant du saccharose avec une saccharose-glucosylmutase.

3. Le procédé selon la revendication 2, dans lequel le mélange de glucides contenant de l'isomaltulose et du saccharose obtenu par conversion enzymatique avec une saccharose-glucosylmutase à partir de saccharose ou d'un mélange initial contenant du saccharose est soumis à une étape de procédé a0) pour réduire la teneur en saccharose à une teneur de 0,01 à 0,50 % en poids de saccharose (en matière sèche par rapport à la matière sèche total du mélange de glucides).

4. Le procédé selon l'une des revendications 1 à 3, dans lequel, dans l'étape de procédé b), une température de réaction de 70 °C à 95 °C, en particulier de 70 °C à 91 °C, est set.

5. Le procédé selon l'une des revendications précédentes, dans lequel, après l'étape de procédé a) et avant l'étape de procédé b), le mélange de glucides fourni à l'étape de procédé a) est préchauffé dans une étape de procédé a1), en particulier à une température de 30 à 80 °C, en particulier de 30 à 75 °C, en particulier de 30 à 70 °C.

6. Le procédé selon l'une des revendications précédentes, dans lequel l'isomaltulose est converti dans l'étape de procédé b) à un taux de conversion de 99 à 100 mol%, en particulier de 99,5 à 100 mol%.

7. Le procédé selon l'une des revendications précédentes, dans lequel l'isomaltulose dans l'étape de procédé b) est converti avec une sélectivité de 97 à 100 mol%, en particulier de 98 à 100 mol%, en 1,6-GPS (6-O-$\alpha$-D-glucopyranosyl-D-sorbitol) et 1,1-GPM (1-O-$\alpha$-D-glucopyranosyl-D-mannitol).

8. Le procédé selon l'une des revendications précédentes, dans lequel le mélange de glucides contenant de l'isomaltulose et du saccharose fourni dans l'étape de procédé a) a 86 à 99,99 % en poids d'isomaltulose, en particulier 98 à 99,99 % en poids d'isomaltulose, et 0,01 à 0,50 % en poids de saccharose (chacun en MS par rapport à la MS totale du mélange de glucides).

9. Le procédé selon l'une des revendications 1 à 7, dans lequel le mélange de glucides contenant de l'isomaltulose et du saccharose fourni dans l'étape de procédé a) a 75,00 à 99,49 % en poids d'isomaltulose, 0,30 à 24,99 % en poids de tréhalulose et 0,01 à 0,50 % en poids de saccharose (chacun en MS par rapport à la MS totale du mélange de glucides).

10. Le procédé selon l'une des revendications 1 à 7 et 9 précédentes, dans lequel le mélange de glucides fourni à l'étape a) a 75 à 86,00 % en poids d'isomaltulose, 0,01 à 0,50 % en poids de saccharose et 13,99 à 24,99 % en poids de tréhalulose (chacun par rapport à la matière sèche du mélange de glucides).

**11.** Le procédé selon l'une des revendications 1 à 10 précédentes, dans lequel la teneur en matière sèche du mélange de glucides présent dans le milieu aqueux est de 35 à 45 % en poids (par rapport au poids total du milieu).

**12.** Le procédé selon l'une des revendications précédentes, dans lequel le catalyseur à base de ruthénium est un catalyseur immobilisé sur un support.

**13.** Le procédé selon l'une des revendications précédentes, dans lequel le catalyseur à base de ruthénium est un catalyseur monométallique ou bimétallique.

**14.** Le procédé selon la revendication 12 ou 13, dans lequel le support est du carbone, un oxyde métallique, en particulier $Al_2O_3$, $TiO_2$, $SiO_2$, $ZrO_2$ ou une zéolite.

**15.** Le procédé selon l'une des revendications précédentes, dans lequel la teneur en ruthénium du catalyseur est de 0,05 à 5,00 % en poids.

**16.** Le procédé selon l'une des revendications précédentes, dans lequel la valeur du pH dans l'étape de procédé b) est entre 2,0 et 5,5.

**17.** Le procédé selon l'une des revendications précédentes, dans lequel la concentration en saccharose dans le mélange de glucides fourni dans l'étape a) est maintenue constante jusqu'à l'isomalt obtenu dans l'étape c) uniquement en réglant les paramètres de procédé définis dans l'étape b).

**18.** Le procédé selon l'une des revendications précédentes, dans lequel l'isomalt obtenu à l'étape c) a au moins 98,00 % en poids de mono- et disaccharides hydrogénés, au moins 86,00 % en poids de 1,6-GPS et 1,1-GPM, 0,01 à 0,50 % en poids de saccharose et au plus 0,30 % en poids de sucres réducteurs (chacun en MS (matière sèche), chacun par rapport à l'isomalt en MS).

**19.** Le procédé selon l'une des revendications précédentes, dans lequel l'isomalt obtenu à l'étape c) a au moins 98,00 % en poids de mono- et disaccharides hydrogénés, au moins 98,00 % en poids de 1,6-GPS et 1,1-GPM, 0,01 à 0,50 % en poids de saccharose, au plus 0,30 % en poids de sucres réducteurs, 0 à 0,50 % en poids de sorbitol et 0 à 0,50 % en poids de mannitol, dans lequel les composants secondaires éventuellement présents dans cet isomalt sont chacun présents en une quantité de 0 à 0,50 % en poids et dans lequel la somme de tous les sucres réducteurs et non réducteurs est au plus de 0,50 % en poids (chacun en MS (matière sèche), chacun par rapport à l'isomalt en MS).

**20.** Le procédé selon l'une des revendications précédentes, dans lequel l'isomalt obtenu à l'étape c) est exempt de sorbitol.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19532396 C2
- DE 19523008 A1
- EP 2361255 B1
- EP 0625578 A1
- EP 0625578 A
- US 4072628 A
- US 3963788 A
- US 4950812 A
- EP 1776015 B1
- DE 69613100 T2
- WO 2005021475 A1
- WO 03104473 A2

**In der Beschreibung aufgeführte Nicht-Patentliteratur**

- **VON JENS HAGEN**. Technische Katalyse - Eine Einführung. VCH Weinheim, 1996